(19) Europäisches Patentamt
European Patent Office
Office européen des brevets

(11) Veröffentlichungsnummer: **0 206 993**
**B1**

(12) **EUROPÄISCHE PATENTSCHRIFT**

(45) Veröffentlichungstag der Patentschrift: **23.05.90**

(21) Anmeldenummer: **86810255.9**

(22) Anmeldetag: **09.06.86**

(51) Int. Cl.$^5$: **C 07 C 309/72**, C 07 D 223/16, C 07 C 227/00, C 07 B 53/00

(54) **Neue Sulfonsäureester.**

(30) Priorität: **13.06.85 CH 2501/85**

(43) Veröffentlichungstag der Anmeldung: **30.12.86 Patentblatt 86/52**

(45) Bekanntmachung des Hinweises auf die Patenterteilung: **23.05.90 Patentblatt 90/21**

(84) Benannte Vertragsstaaten: **AT BE CH DE FR GB IT LI NL SE**

(56) Entgegenhaltungen:
**EP-A-0 050 850**
**EP-A-0 081 320**
**EP-A-0 126 986**
**EP-A-0 134 392**
**EP-B-0 072 352**
**US-A-4 473 575**

**ANGEWANDTE CHEMIE, 95. Jahrgang, Heft 1, 1983, Weinheim F. EFFENBERGER et al. "Trifluormethansulfonate von alpha-Hydroxycarbonsäureestern-Edukte zur racemisierungsfreien Synthese H-substituierter alpha-Aminosäuren"**

(73) Patentinhaber: **CIBA-GEIGY AG**
**Klybeckstrasse 141**
**CH-4002 Basel (CH)**

(72) Erfinder: **Blaser, Hans-Ulrich, Dr.**
**Brücklismattstrasse 18**
**CH-4107 Ettingen (CH)**
Erfinder: **Jalett, Hans-Peter**
**Sevogelstrasse 70/3**
**CH-4052 Basel (CH)**
Erfinder: **Sedelmeier, Gottfried, Dr.**
**Erlenweg 11**
**D-7801 Schallstadt (DE)**

**EP 0 206 993 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue Sulfonsäureester, Verfahren zu ihrer Herstellung sowie die Verwendung dieser Verbindungen als Zwischenprodukte bei der Herstellung von ACE-Inhibitoren oder deren Vorstufen.

Die neuen Sulfonsäureester haben die Formel I,

$$R^1-CH_2-CH_2-\underset{*}{\overset{COOR^2}{\overset{|}{C}H}}-OSO_2-R^3 \qquad (I)$$

worin $R^1$ für unsubstituiertes oder durch $C_1$—$C_7$-Alkyl substituiertes $C_5$—$C_6$-Cycloalkyl, oder für unsubstituiertes oder substituiertes Phenyl steht, $R^2$ $C_1$—$C_7$-Aklyl bedeutet, $R^3$ für Phenyl steht, das durch Halogen oder Nitro substituiert ist, und das Symbol * ein Kohlenstoffatom kennzeichnet, das entweder bei der überwiegenden Zahl von Molekeln in S- oder bei der überwiegenden Zahl von Molekeln in R-Konfiguration vorliegt.

Ein Phenylrest $R^1$ kann z.B. durch Substituenten aus der Gruppe $C_1$—$C_7$-Alkyl, z.B. Methyl, Hydroxy, $C_1$—$C_7$-Alkoxy, z.B. Methoxy, $C_1$—$C_7$-Alkanoyloxy, z.B. Acetoxy, Fluor, $C_1$—$C_7$-Alkylendioxy, z.B. Ethylendioxy, Amino, $C_1$—$C_7$-Alkylamino, z.B. Methylamino, Di-($C_1$—$C_7$)-alkylamino, z.B. Dimethylamino, $C_1$—$C_7$-Alkanoylamino, z.B. Acetylamino, Carbamoyl, $C_1$—$C_7$-Alkycarbamoyl, z.B. Methylcarbamoyl, Di-($C_1$—$C_7$)-alkycarbamoyl, z.B. Dimethylcarbamoyl, $C_1$—$C_7$-Alkansulfonylamino, z.B. Methan- oder Ethansulfonylamino, Sulfamoyl, $C_1$—$C_7$-Alkylsulfamoyl, z.B. Methylsulfamoyl, Di($C_1$—$C_7$)-alkylsulfamoyl, z.B. Dimethylsulfamoyl, Halogen-$C_1$—$C_7$-alkyl, z.B. Trifluormethyl, Hydroxy-$C_1$—$C_7$-alkyl, z.B. Hydroxymethyl, und Amino-$C_1$—$C_7$-alkyl, z.B. Aminomethyl oder 2-Aminoethyl, substituiert sein.

Ver- und nachstehend haben die verwendeten Allgemeinbegriffe die folgenden Bedeutungen: Das Präfix "$C_1$—$C_7$" kennzeichnet organische Reste mit 1 bis 7 und insbesondere 1 bis 4 Kohlenstoffatomen.

$C_1$—$C_7$-Alkyl, ist z.B. Methyl, Ethyl, Propyl, Isopropyl, Butyl oder tert-Butyl, kann aber auch Pentyl, Hexyl oder Heptyl sein.

$C_1$—$C_7$-Alkoxy ist z.B. Methoxy, Ethoxy, Propoxy, Isopropoxy oder einer der vier isomeren Butoxyreste.

$C_1$—$C_7$-Alkanoyl ist z.B. Formyl, Acetyl, Propionyl oder Butyryl, ferner Isobutyryl oder Pivalolyl.

$C_1$—$C_7$-Alkanoyloxy ist z.B. Acetoxy, Propionyloxy, Butyryloxy, ferner Formyloxy oder Pivaloyloxy.

$C_5$—$C_6$-Cycloalkyl ist Cyclopentyl oder Cyclohexyl. Durch $C_1$—$C_7$-Alkyl substituiertes $C_5$—$C_6$-Cycloalkyl ist z.B. Ethyl- oder Methylcyclohexyl, wie 4-Methylcycloahexyl.

Aryl ist z.B. Naphthyl und insbesondere Phenyl.

1-Aryl-$C_1$—$C_7$-alkyl ist z.B. 1-Naphthylethyl, Benzyl oder insbesondere 1-Phenylethyl.

Halogen ist z.B. Fluor oder Iod, insbesondere aber Chlor oder Brom.

$C_1$—$C_7$-Alkylendioxy ist z.B. Ethylendioxy, 1,3-Propylendioxy, 2,3-Butylendioxy oder 1,3(2,2-Dimethyl)propylendioxy.

$C_1$—$C_7$-Alkylamino oder Di-($C_1$—$C_7$)-alkylamino ist z.B. Methylamino, Dimethylamino, Ethylamino, Diethylamino, Propylamino, Isopropylamino oder Butylamino.

$C_1$—$C_7$-Alkanoylamino ist z.B. Acetylamino oder Propionylamino, ferner Formylamino.

$C_1$—$C_7$-Alkylcarbamoyl oder Di-($C_1$—$C_7$)-alkylcarbamoyl ist z.B. Methylcarbamoyl, Dimethylcarbamoyl, Ethylcarbamoyl, Diethylcarbamoyl, Propylcarbamoyl oder Butylcarbamoyl.

$C_1$—$C_7$-Alkansulfonylamino ist z.B. Methansulfonylamino, Ethyansulfonylamino oder Propansulfonylamino.

$C_1$—$C_7$-Alkysulfamoyl oder Di-($C_1$—$C_7$)-alkylsulfamoyl ist z.B. Methylsulfamoyl, Dimethylsulfamoyl, Ethylsulfamoyl, Diethylsulfamoyl Propylsulfamoyl oder Butylsulfamoyl.

Halogen-$C_1$—$C_7$-alkyl ist z.B. Halogenmethyl, wie Trifluormethyl, oder 2-Chlorethyl.

Hydroxy-$C_1$—$C_7$-alkyl ist z.B. Hydroxymethyl oder 1- und in erster Linie 2-Hydroxyethyl.

Amino-$C_1$—$C_7$-alkyl ist z.B. Aminomethyl oder 1- oder 2-Aminoethyl.

Durch Nitro substituiertes Phenyl $R^3$ ist z.B. Mono- oder Dinitrophenyl, wie 2-, 3- oder 4-Nitrophenyl oder 2,4-Dinitrophenyl.

Durch Halogen substituiertes Phenyl $R^2$ ist ein- bis fünffach durch Halogen, wie Fluor, Chlor oder Brom, substituiertes Phenyl und bedeutet z.B. Bromphenyl, Trichlorphenyl oder Pentafluorphenyl.

Das Symbol *, das ein Kohlenstoffatomm kennzeichnet, das bei der überwiegenden Zahl von Molekeln in S- oder bei der überwiegenden Zahl von Molekeln in R-Konfiguration vorliegt, beziechnet den Umstand, dass die Verbindungen der Formel I bezüglich dieses Kohlenstoffatoms nicht als Racemate, sondern als überwiegend reine Enantiomere erhalten werden. Der Begriff "überwiegend rein" bedeutet im Zusammenhang mit Formel I eine von der 50:50-Verteilung eines Racemats dadurch abweichende Enantiomerenverteilung, dass sie zumindest 90:10, bevorzugt zumindest 95:5 und insbesondere 98:2 bis 100:0 zugunsten der R- oder der S-Form beträgt. Dabei sind Verbindungen der Formel I mit einem wie vorstehend definiert überwiegenden R-Anteil bevorzugt.

Bevorzugt sind ferner Verbindungen der Formel I, worin $R^1$ für $C_5$—$C_6$-Cycloalkyl, Phenyl oder für durch $C_1$—$C_7$-Alkyl, Hydroxy, $C_1$—$C_7$-Alkoxy, $C_1$—$C_7$-Alkanoyloxy, Fluor, Trifluormethyl oder $C_1$—$C_7$-Alkylendioxy substituiertes Phenyl steht, und $R^2$, $R^3$ und * die vorstehend angegebenen Bedeutungen aufweisen.

Weiter bevorzugt sind Verbindungen der Formel I, worin $R^1$ für $C_5$—$C_6$-Cycloalkyl, Phenyl oder durch einen der Reste $C_1$—$C_4$-Alkyl, Hydroxy, $C_1$—$C_4$-Alkoxy oder Fluor substituiertes Phenyl steht, $R^2$ $C_1$—$C_4$-Alkyl bedeutet, $R^3$ für 2-, 3- oder 4-Nitrophenyl, 2,4-Dinitrophenyl oder für Pentafluorphenyl steht und das Symbol * ein Kohlenstoffatom kennzeichnet, das entweder bei der überwiegenden Zahl von Molekeln in S- oder bei der überwiegenden Zahl von Molekeln in R-Konfiguration vorliegt, insbesondere jedoch in R-Konfiguration.

Besonders bevorzugt sind Verbindungen der Formel I, worin $R^1$ für Cyclohexyl, Phenyl, $C_1$—$C_4$-Alkylphenyl oder $C_1$—$C_4$-Alkoxyphenyl steht, $R^2$ $C_1$—$C_4$-Alkyl bedeutet, $R^3$ für 2-Nitrophenyl oder 2,4-Dinitrophenyl steht und das Symbol * ein Kohlenstoffatom kennzeichnet, das bei der überwiegenden Zahl von Molekeln in R-Konfiguration vorliegt.

Insbesonders bevorzugt sind Verbindungen der Formel I, worin $R^1$ für Phenyl steht, $R^2$ $C_1$—$C_4$-Alkyl bedeutet, $R^3$ für 4-Nitrophenyl oder 2,4-Dinitrophenyl steht und das Symbol * ein Kohlenstoffatom kennziechnet, das bei der überwiegenden Zahl von Molekeln in R-Konfiguration vorliegt; unter diesen vor allem diejenigen Verbindungen, in denen $R^2$ Ethyl bedeutet.

Die Verbindungen der Formel I lassen sich auf an sich bekannte Weise herstellen, indem man einen α-Hydroxyester der Formel II,

$$R^1-CH_2-CH_2-\overset{*}{C}H(COOR^2)-OH \qquad (II)$$

worin $R^1$ und $R^2$ wie für Formel I definiert sind und * ein Kohlenstoffatom kennzeichnet, das entweder bei der überwiegenden Zahl von Molekeln in S- oder bei der überwiegenden Zahl von Molekeln in R-Konfiguration vorliegt, mit einer den Substituenten —OH in den Rest der Formal —$OSO_2$—$R^3$ überführenden Verbindung umsetzt, worin $R^3$ wie für Formel I definiert ist.

Den Substituenten —OH in den Rest der Formel —$OSO_2$—$R^3$ überführende Verbindungen sind z.B. $R^3$-Sulfonsäureanhydride, wie gemischte Anhydride, z.B. mit Halogenwasserstoffsäuren, d.h. $R^3$ Sulfonsäurehalogenide, wie -chloride oder -bromide, sowie Anhydride der jeweiligen $R^3$-Sulfonsäuren selbst, d.h. Verbindungen vom Typus $R^3$—$SO_2$—O—$SO_2$—$R^3$. Die Umsetzung wird vorteilhaft in einem inerten Lösungsmittel sowie in Gengenwart einer Base durchgeführt. Als Lösungsmittel sind z.B. halogenierte Kohlenwasserstoffe, wie Dichlormethan, Chloroform oder Tetrachlorkohlenstoff, geeignet, ferner Kohlenwasserstoffe, wie Toluol, Benzol oder Hexan. Als Basen können anorganische oder organische Basen verwendet werden, z.B. basische Erdalkali- oder Alkalimetallsalze, wie Alkalimettalcarbonate, z.B. Kaliumcarbonat, Natriumcarbonat, Natriumhydrogencarbonat, ferner z.B. tertiäre Amine, wie Pyridin, oder Trialkylamine, wie Triethylamin.

Die Umsetzung wird zweckmässigerweise bei Temperaturen zwischen −50° und +110°C, gegebenenfalls unter Schutzgas, wie unter Stickstoff oder Argon, durchgeführt.

Die Unsetzung verläuft stereochemisch einheitlich in der Weise, dass die Konfiguration am mit * gekennzeichneten Kohlenstoffatom erhalten bleibt. Daher bedeutet das Symbol * im Zusammehang mit Formel II eine Enantiomerenverteilung in der Abstufung, die bei Formel I definiert ist, zumindest aber eine Enantiomerenverteilung von 80:20, bevorzugt von zumindest 85:15.

Ausgangsverbindungen der Formel II sind bekannt oder lassen sich auf bekannte Weise herstellen. Zahlreich bekannte Herstellungsverfahren (siehe z.B. Europäische Patentanmeldung 126 986), wie die Reduktion entsprechender α-Ketoester mit Raney-Nickel und Wasserstoff oder die saure Verseifung entsprechender α-Hydroxynitrile mit anschliessender Veresterung, führen zu racemischen α-Hydroxyestern, die in einem nachgeschalteten Schritt, z.B. über Diastereomere, chromatographisch oder auf dem Weg der fraktionierten Kristallisation, getrannt werden müssen. Dieser Trennschritt bringt regelmässig den Verlust von mindestens 50% der in die Trennung eingebrachten Sunstanz mit sich. Es besteht daher das Bedürfnis nach einen Verfahren, das eine derartige verlustreiche Isomerentrennung vermeidet.

Im Rahmen der vorliegenden Erfindung konnte festgestellt werden, dass sich ein zur asymmetrischen Reduktion von gewissen α-Ketoestern (siehe z.B. US—Patentschrift 4,329,487 oder japanische Offenlegungeschrift 80 35,060) bekanntes verfahren mit gutem Erfolg auf Verbindungen der Formel III,

$$R^1-CH_2-CH_2-C(COOR^2)=O \qquad (III)$$

worin $R^1$ und $R^2$ wir für Formel I definiert sind, übertragen lässt. Ein weiterer Gegenstand der Erfindung

besteht daher darin, dass man eine Verbindung der Formel I, die vie vorstehend definiert ist, herstellt, indem man eine Verbindung der Formal III,

$$R^1-CH_2-CH_2-\underset{}{\overset{COOR^2}{\overset{|}{C}}}=O \qquad (III)$$

worin $R^1$ und $R^2$ wie für Formel I definiert sind, in Gegenwart eines Platinkatalysators auf einem Träger sowie eines Cinchonaalkaloides zu einer Verbindung der Formel II, die wie vorstehend definiert ist, enantioselektiv reduziert,

$$R^1-CH_2-CH_2-\underset{*}{\overset{COOR^2}{\overset{|}{C}}}H-OH \qquad (II)$$

und die erhaltene Verbindung der Formel II mit einer den Substituenten —OH in den Rest der Formel $—OSO_2—R^3$ überführenden Verbindung umsetzt, worin $R^3$ wie für Formel I definiert ist.

Die Reduktion einer Verbindung der Formel III wird als enantioselektiv bezeichnet, wenn die optische Ausbeute 60% oder mehr, bevorzugt 70% oder mehr, insbesondere 80% oder mehr, beträgt. Das überwiegende Vorliegen einer Konfiguration am mit * gekennzeichneten Kohlenstoffatom bezieht sich im Zusammenhang mit Formel II daher auf Enantiomerenverteilungen von zumindest 80:20, bevorzugt zumindest 85:15 und besonders bevorzugt zumindest 90:10m zugunsten der R- oder S-Form.

Die enantioselektive Reduktion wird auf an sich bekannte Weise durchgeführt. Die verwendeten Platinkatalysatoren sind auf inerten Trägern aufgebracht, wie z.B. auf Koble, Aluminiumoxid, Calciumcarbonat oder Bariumsulfat, bevorzugt auf Aluminiumoxid. Die Katalysatoren werden auf bekannte Weise mit Wasserstoff bei 200—400°C aktiviert und anschliessend mit der Lösung eines Cinchonaalkaloides modifiziert (imprägniert) und/oder ein Cinchonaalkaloid wird bei der Reduktion direkt zugesetzt. Unter Cinchonaalkaloiden wird die auch Chinaalkaloide gennante Gruppe von Chinolin-Pflanzenbasen verstanden, die vornehmlich aus der Rinde von Bäumen der Gattung Cinchona und Remijia isoliert werden können. Insbesonders fallen darunter die Alkaloide (−)-Chinin, (+)-Chinidin, (+)-Cinchonin und (−)-Cinchonidin. Dabei führt die Verwendung von (−)-Chinin und (−)-Cinchonidin zu Verbindungen der Formel II in der R-Form, während bei der Verwendung von (+)-Chinidin und (+)-Cinchonin Verbindungen der Formel II in der S-Form erhalten werden. Die Verwendung von (−)-Chichonidin ist bevorzugt. Die Hydrierung findet zweckmässigerweise in einem Druckreaktor, wie einem Autoklaven, bei einem Wasserstoffdruck von 10 bis 170 bar, insbesondere von 50 bis 150 bar, und bei Temperaturen von Raumtemperatur +30°C, insbesondere bei 0° bis 30°C, statt. Bevorzugte Lössungsmittel für die Imprägnierung sind solche, die das verwendete Chinaalkaloid lösen, insbesondere, $C_1—C_7$-Alkanole, wie Ethanol, oder Ether, wie Tetrahydrofuran. Für die Hydrierung geeignete Lösungsmittel sind z.B. aromatische Kohlenwasserstoffe, wie Benzol oder Toluol, ferner halogenierte Kohlenwasserstoffe, wie Dichlormethan, Ether, wie tert.-Butylmethylether, oder niedrigsiedende Carbonsäureester, wie Essigester.

Wie der Definition des Symbols * in Zussamenhang mit Formel II entnommen werden kenn, lassen sich die Verbindungen der Formel II auf vorstehend beschriebene Weise in optischen Ausbeuten von zumindest 60%, bevorzugt 70% und insbesondere 80%, erhalten. Im Zuge der weiteren Umsetzung zu Sulfonsäureestern der Formel I, wie vorstehend beschrieben, lässt sich die optische Ausbeute dahingehend steigern, dass Verbindungen der Formel I überwiegend rein erhalten werden, d.h. in optischen Ausbeuten von zumindest 95%, bevorzugt zumindest 97,5% und insbesondere 99 bis 100%.

Die Verbindungen der Formel I sind wertvolle Zwischenprodukte bei der Herstellung von ACE-Inhibitoren oder daren Vorstufen. Diese Wirkstoffklasse had in den vergangenen Jahren wechsendes Interesse gefunden. Sie erweitert das Potential dar verfügbaren Anti-hypertensive und damit die therepeutischen Möglichkeiten bei der Bekämpfung des Bluthochdrucke. Bei einer Reihe von wirksamen ACE-Inhibitoren (siehe z.B. europäische Patentanmeldungen 50 850 und 72 352) ist das Strukturelement der Teilformel IV bedeutsam,

$$R^1-CH_2-CH_2-\underset{*}{\overset{COOR^2}{\overset{|}{C}}}H-NH- \qquad (IV)$$

worin $R^1$ und $R^2$ wie für Formel I definiert sind und das Symbol * ein Kohlenstoffatom bezeichnet, das in der S-Konfiguration vorliegt.

Die Bindung zwischen dem Stickstoffatom der Teilformel IV und dem benachbarten Kohlenstoffatom der Teilformel IV ist nach den bisher bekanngewordenen Verfahren z.B. geknüpft worden, indem eine

Verbindung der Formel III unter reduktiven Alkylierungsbedingungen mit einem primären oder sekundären Amin ungesetzt wird (Reaktion 1)

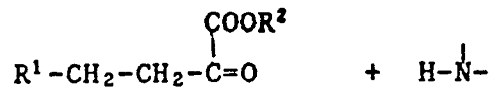 (Reaktion 1)

(III)

oder indem ein α-Bromester der Formel Va eingesetzt wird (Reaktion 2),

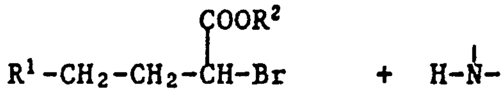 (Reaktion 2)

(Va)

oder indem eine ungesättigte Verbindung der Formel Vb eingesetzt wird (Reaktion 3),

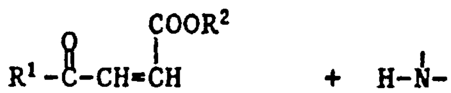 (Reaktion 3)

(Vb)

wobei jeweils $R^1$ und $R^2$ wie vorstehend definiert sind.

Nach den Verfahren nach Reaktion 1 und 2 können die gewünschten Verbindungen der S-konfiguriertern Strukter der Teilformel IV auf direktem Wege nicht erhalten werden. Stattdessen muss des erhaltene racemische Material einer Racematspaltung unterworfen werden, die zum Verlust von zumindest 50% des eingesetzten Materials führt. Da das Stickstoffatom der Teilformel IV zu dem Zeitpunkt, zu dem die Unsetzung nach Reaktion 1 oder Reaktion 2 stattfindet, zumeist bereits seinerseite Bestandteil eines komplexion chiralen Moleküls ist, kann ein Substanzverlust von zumindest 50% in dieser späten Phase der Gesamtsynthese eines ACE-Hemmers nicht befriedigen.

Nach dem Verfahren nach Reaktion 3 kann zwar eine etwas günstigeres Isomerenverhältnis erzielt werden, als es ein Racemat aufweist, nämlich bis zu 2:1; es muss sich aber dann noch ein weiterer Reaktionsschrift, eine Reduktion, anschliessen.

Die erfindungsgemässe Verwendung der Verbindungen der Formel I ungeht die vorstehend beschriebenen Nachteile. Danach können nämlich, ausgehend von Verbindungen der Formel III, Verbindungen, die die Teilformel IV enhalten, in chemischen Ausbeuten von mehr als 50% erhalten werden.

Im einzelnen beruht diese vorteilhafte Verwendung unter anderem darauf, dass sich die Verbindungen der Formel I ohne nennenswerte Racemisierung oder das Auftreten von Eliminierungsprodukten mit primären oder sekundären Aminen unsetzen lassen. So werden in hohen chemischen und optischen Ausbeuten unter Inversion Verbindungen mit der Teilformel IV erhalgen, wenn Verbindungen der Formel I eingesetzt werden, in denen * ein Kohlenstoffatom kennzeichnet, das bei der überwiegenden Zahl von Molekeln in der R-Konfiguration vorliegt. Ebenso lassen sich, ausgehend von Verbindungen der Formel I, in denen * Kohlenstoffatom kennzeichnet, das bei der überwiegenden Zahl von Molekeln in der S-Konfiguration vorliegt, Verbindungen mit einer der Teilformel IV entsprechenden Strukter erhalten, in der das Symbol * ein Kohlenstoffatom kennzeichnet, das in der R-Konfiguration vorliegt.

Dies ist unerwartet und überraschend, da nach dem Stand der Technik mit Nebenreaktionen sowie mit Racemisierung gerechnet werden muss. Beides würde die chemischen Ausbeuten deutlich unter 50% absinken lassen.

Zwar beachreiben bereits F. Effenberger et al., Angew. Chem. *95*, 50 (1983), eine Abgangsgruppe, die zur racemisierungsfreien Synthese N-substituierter α-Aminosäuren, ausgehend von α-Hydroxycarbonsäureestern, geeignet ist. Es handelt sich dabie um die α-Trifluormethansulfonyloxy-Gruppe. In derselben Veröffentlichung raten aber die Autoren von der Verwendung anderer Abgangsgruppen ab, da bei α-Methansulfonyloxy- und α-Toluolsulfonyloxy-carbonsäurederivaten- infolge drastischer Reaktionsbedingungen — unter anderem Racemisierungs- und Eliminierungsprodukte auftretren. Ferner liegen die Ausbeuten bei Verwendung von α-Brom-, α-Methansulfonyloxy-, α-Toluolsulfonyloxy- und α-Chlorpropionsäureethylester nach 22 Stunden bei 40, 10, 5 bzw. 1%, während der Umsatz der vorgeschlagenen α-Trifluoromethansulfonyloxy-Verbindung nach 20 Minuten 100% beträgt.

Abgesehen von dem danach grundsätzlich nich zu erwartenden Resultat, dass aromatische

Sulfonyloxyverbindungen ausgezeichnet zur racemisierungsfrein Synthese von α-Aminosäuren geeignet sind, weisen die erfindungsgemäss verwendeten Verbindungen mit dem Rest $R^2$ gegenüber den nach dem Stand der Technik bekannten Verbindungen mit der Abgangsgruppe $CF_3SO_2$—O— nachhaltige Vorteile auf. So sind sie deutlich billiger, besser umweltverträglich und sehr viel weniger toxisch.

Ein weiterer Aspekt der vorliegenden Erfindung besteht daher in der Verwendung der Verbindungen der Formel I zur Herstellung von Verbindungen der Formel VI,

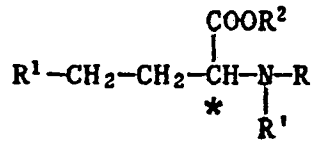

(VI)

dadurch gekennzeichnet, dass man eine Verbindung der Formel VII

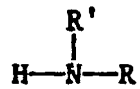

(VII)

unter Inversion mit einer Verbindung der Formel I

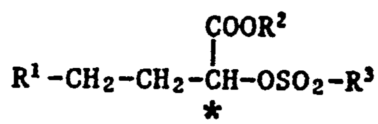

(I)

alkyliert, wobei $R^1$, $R^2$ und $R^3$ wie vorstehend definiert sind, das Symbol * ein Kohlenstoffatom kennzeichnet, das entweder bei der überwiegenden Zahl von Molekeln in S- oder bei überwiegenden Zahl von Molekeln in R-Konfiguration vorliegt, und wobei R′ Wasserstoff oder $C_1$—$C_7$-Alkyl ist und R den Rest der Teilformel VIII

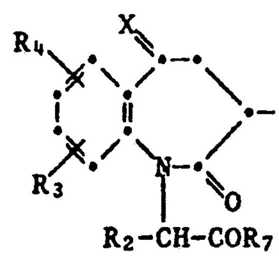

(VIII)

bedeutet, wobei in dieser Teilformel VIII die Reste $R_2$, $R_3$, $R_4$, $R_7$ (mit tiefgestellten Indizes) und X die Bedeutungen haben, die in der europäischen Patentanmeldung 72 352 definiert sind, oder wobei R′ Wasserstoff oder $C_1$—$C_7$-Alkyl ist und R den Rest der Teilformel IX

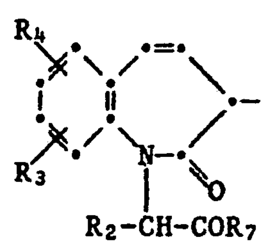

(IX)

bedeutet, wobei in dieser Teilformel IX die Reste $R_2$, $R_3$, $R_4$ und $R_7$ die Bedeutungen haben, die in der europäischen Patentanmeldung 72 352 definiert sind, oder wobei R′ für Wasserstoff und R für 1-Aryl-$C_1$—$C_7$-alkyl steht, oder wobei R′ und R Wasserstoff bedeuten.

Die Bedeuten der Substituenten der Teilformel VIII oder IX gemäss der europäischen Patentanmeldung 72 352 sind Wasserstoff oder niedrig-Alkyl für $R_2$, für $R_3$ und $R_4$ unabhängig voneinander Wasserstoff, niedrig-Alkyl, niedrig-Alkoxy, niedrig-Alkanoyloxy, Hydroxy, Halogen oder Trifluoromethyl, oder $R_3$ und $R_4$ bedeuten gemeinsam niedrig-Alkylendioxy, $R_7$ bildet gemeinsam mit der Carbonylgruppe, an die es

gebunden ist, Carboxy oder ein funktionell modifiziertes Carboxy $COR_7$, und X steht für Oxo, zwei Wasserstoffatome oder ein Wasserstoffatom gemeinsam mit einer Hydroxygruppe. Dabei bezeichnet der Begriff "niedrig" organische rest bzw. Verbindungen, die bis zu 7, vorzugsweise bis zu 4 und vorteilhafterweise 1 oder 2 Kohlenstoffatome enthalten. Für weitergehende Definitionen wird auf die erwähnte Offenlegungsschrift verwiesen.

Die vorstehende Umsetzung, eine substitutive Alkylierung, wird unter herkömmlichen allgemeinen Bedingungen bei Temperaturen im Bereich von etwa 0° bis zur Siedetemperatur der Reaktionsmischung, vorzugsweise bei Temperaturen zwischen Raumtemperatur und etwa 100°C, durchgeführt. Die Umsetzung findet vorteilhaft in Gegenwart eines Lösungsmittels, das im Hinblick auf die Reaktanten inert ist, statt, wie in Gegenwart eines chlorierten Niederalkans (z.B. Chloroform oder Methylenchlorid), eines acyclischen oder cyclischen Ethers (z.B. Diethylether, 1,2-Dimethoxyethan, Dioxan oder Tetrahydrofuran), eines Niederalkancarbonitrils (z.B. Acetonitril), eines leichtflüchtigen Niederalkancarbonsäureniederalkylesters (z.B. Essigester) oder eines tertiären Amids mit niedrigem Molekular- gewicht (z.B. N,N-Dimethylformamid, N,N-Dimethylacetamid, N-Methylpyrrolidon, N-Ethylpiperidon und Hexamethylphosphorsäuretrisamid). Vorteilhafterweise wird die während der Reaktion freigesetzte starke Säure $HOSO_2$—$R^3$ durch Zugabe eines säurebindenden Mittels gebunden, wie vorzugsweise eines anorganischen Säurfängers, wie eines Alkalimetallbicarbonats, -carbonats oder -hydroxids, eines organischen quaternären Ammoniumsalzes (z.B. eines Tetrabutylammoniumsalzes) oder einer organischen tertiären Base, wie Triethylamin, N-Ethylpiperidin, N-Methylmorpholin, Pyridin oder Chinolin.

Diese Umsetzung verläuft unter Inversion, d.h. stereochemisch einheitlich in der Weise, dass die Konfiguration am mit * gekennzeichneten Kohlenstoffatom sich unkehrt. Wenn daher in einer Verbindung der Formel I das Symbol * ein Kohlenstoffatom kennzeichnet, das bei der überweigenden Zahl von Molekeln in R-Konfiguration vorliegt, kennzeichnet das Symbol * in der aus dieser Verbindung I entstehenden Verbindung der Formel VI ein Kohlenstoffatom, das bei der überwiegenden Zahl von Molekeln in S-Konfiguration vorliegt, und vice versa. Der Begriff "überwiegend in einer Konfiguration vorliegend" hat im Zusammenhang mit Formel VI die auch schon für Formel I angegebene Bedeutung. Für den Fall, dass in der Formel VII R und R' Wasserstoff bedeuten, wenn es sich daher im die Unsetzung einer Verbindung der Formel I mit Ammoniak handelt, wird die Reaktion vorzugsweise unter erhöltem Druck, z.B. bei 10 bis 20 bar, in einem inerten Lösungsmittel, wie Acetonitril, durchgeführt.

Die Ausgangsverbindungen der Formel VII sind bekannt oder können auf an sich bekannte Weise hergestellt werden (siehe z.B. europäische Patentanmeldung 72 352). Die erhaltenen Verbindungen der Formel VI sind entweder ACE-Hemmer der Formel VIa,

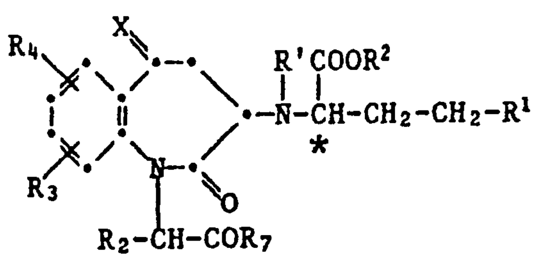

(VIa)

worin $R^1$, $R^2$, R', * sowie $R_2$, $R_3$, $R_4$, $R_7$ und X wie vorstehend definiert sind, oder es sind Vorstufen zu ACE-Hemmern der Formel VIb,

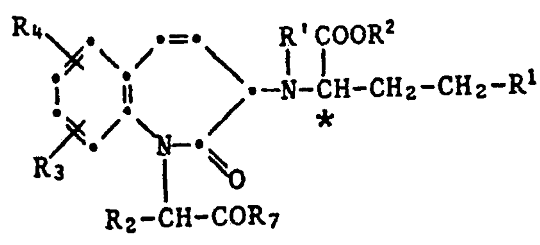

(VIb)

worin $R^1$, $R^2$, R', * sowie $R_2$, $R_3$, $R_4$, $R_7$ wie vorstehend definiert sind, oder es sind Vorstufen zu ACE-Hemmern der Formel VIc,

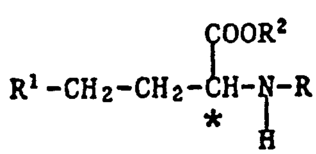

(VIc)

worin $R^1$, $R^2$ und * wie vorstehend definiert sind und R für 1-Aryl-$C_1$—$C_7$-alkyl, z.B. für Benzyl, 1-Phenylethyl

oder 1-Naphthylethyl, steht, oder, wenn auch R für Wasserstoff steht, sind es Vorstufen zu ACE-Hemmern der Formel VId

$$R^1-CH_2-CH_2-\overset{\overset{\displaystyle COOR^2}{|}}{\underset{*}{CH}}-NH_2 \qquad (VId)$$

worin $R^1$, $R^2$ und * wie vorstehend definiert sind.

Verbindungen der Formel VIb lassen sich auf an sich bekannte Weise durch Reduktion (Hydrogenolyse) in Verbindungen der Formel VIa, worin X für zwei Wasserstoffatome steht, überführen. Dieses Verfahren erweist sich als besonders vorteilhaft und ist daher bevorzugt, wenn in einer Verbindung der Formel VIb $R_7$ für 1-Aryl-$C_1$—$C_7$-Alkoxy, z.B. für Benzyloxy, steht. In einem solchen Fall lassen sich nämlich die Reduktion der C-C-Doppelbindung und die Ueberführung einer beispielsweise Benzyloxycarbonylgruppe $COR_7$ in die Carboxygruppe $COR_7$ gleichzeitig durchführen.

Verbindungen der Formel VId sind ausser auf direktem Wege (Verbindung der Formel I + Ammoniak) ohne nachteilige Auswirkungen auf die chemische oder optische Ausbeute auch auf einem zweistufigen Weg erhältlich. So lassen sich Verbindungen der Formel VIc auf an sich bekannte Weise unter schonenden Bedingungen (Hydrogenolyse) und unter Erhalt der Konfiguration an mit * gekennzeichneten Kohlenstoffatom in Verbindungen der Formel VId überführen.

Die Aminosäreester der Formel VId sind ihrerseits geeignet, als wesentliche Bausteine bei der Synthese von ACE-Hemmern zu dienen, da sie das als bedeutsam erkannte Strukturelement der Teilformel IV in sich tragen.

Zusammenfassend erweisen sich die neuen Verbindungen der Formel I als Schlüsselverbindungen auf dem Weg zu ACE-Hemmern, sei es auf dem direkten Weg von I zu VIa, auf dem Weg von I über VIb zu VIa oder auf dem Weg von I ausgehend über VId, wobei dabei wahlweise Verbindungen der Formel VIc durchlaufen werden können. Dabei zeichnet sich die erfindungsgemässe Synthese durch hohe chemische und optische Ausbeuten aus.

Die nachfolgenden Beispiele sollen die vorgliegende Erfindung erläutern, ohne sie, etwa auf den Umfang der Beispiele, zu beschränken. Die mit dem Zusatz "ee" versehenen Prozentangaben sind optische Ausbeuten. Temperaturen sind in Celsius-Graden angegeben.

Beispiel 1

Die Vorbereitung des Katalysators und die Durchführung der Hydrierung kann analog zu den Angaben im US—Patent 4,329,487 erfolgen:

Vorbereitung des Katalysators: 1 g 5% Pt/C (z.B. Degussa Typ D 101 R) wird 3 Stunden unter einem leichten Wasserstoffstrom auf 300°C erhitzt. Nach Abkühlen unter Argon wird der Katalysator in 80 ml einer 1% ethanolischen Cinchonidinlösung unter Rückfluss gekocht, abfiltriert, mit wenig Ethanol und danach mit dem bei der Hydrierung verwendung Lösungsmittel gewaschen.

Durchführung der Hydrierung: 20 g 4-Phenyl-2-oxo-buttersäureethylester werden in 100 ml Benzol gelöst und in einen 300 ml Autoklaven gespült, der mit einem Begasungsrührer ausgestattet ist. Anschliessend werden 0,1 g Cinchonidin und der vorbereitete Katalysator zugegeben, und die Hydrierung wird auf übliche Weise bei 150 bar Gesamtdruck und 20—30°C durchgeführt. Nach Beendigung der Wasserstoffaufnahme wird der Katalysator abfiltriert und das Lösungsmittel am Rotationsverdampfer entfernt. Die chemische Ausbeute an 2-Hydroxy-4-phenyl-buttersäureethylester beträgt ca: 95%, die optische Ausbeute an der R-Form beträgt 70%

Beispiel 2

Es wird wie in Biespiel 1 vorgegangen, jedoch wird 1 g 5% Pt/$Al_2O_3$ (z.B. Engelhard Typ 4759 b) verwendet, und die Wasserstoffbehandlung wird 2 Stunden bei 400°C durchgeführt. Die chemische Ausbeute beträgt ca. 95%, die optische Ausbeute 72%.

Biespiel 3

Es wird wie in Beispiel 2 vorgegangen, jedoch wird der Katalysator nicht mit einer Cinchonidinlösung vorbehendelt. Die chemische Ausbeute beträgt ca. 95%, die optische Ausbeute 68%.

Beispiel 4

Es wird wie in Biespiel 2 vorgegangen, dabei werden für die Hydrierung folgende Lösungsmittel verwendet: a) Toluol, b) Dichlormethan, c) Essigester, d) t-Butyl-methylether. Die chemischen Ausbeuten betragen ca. 95%, die optischen Ausbeuten liegen zwischen 60 und 70%.

Beispiel 5

Die Reaktion wird analog zu Biespiel 3, jedoch bei einer Reaktionstemperatur von 5°C durchgeführt. Die chemische Ausbeute beträgt ca. 95%, die optische Ausbeute 80%.

Biespiel 6

104,16 g (−)-R-2-Hydroxy-4-phenyl-buttersäureethylester vom Drehwert $[\alpha]_D^{20} = 17,0°$ (82% ee) und 121,89 g 4-Nitrobenzolsulfonylchlorid werden bei Raumtemperatur in 500 ml Toluol gelöst. Bei einer Innentemperatur von 0°C werden innerhalb einer Stunde 66,8 g Triethylamin zugetropft. Anschliessend wird noch 1 Stunde bei Raumtemperatur gerührt. Nach wässriger Aufarbeitung und Extraktion der Toluolphase mit 1 N Salzsäure werden die vereinigten Toluolphasen über wenig Kieselgel filtriert und am Ratationsverdampfer eingeengt. Das zurückbleibende Oel wird in 100 ml Cyclohexan/Essigester (4:1) aufgenommen und 48 Stunden bei Raumtemperatur, anschliessend 8 Stunden bei 0°C, gerührt und dann filtriert. Der Filterrückstand wird getrocknet und ergibt 41,5 g racemischen 2-(4-Nitrobenzolsulfonyloxy)-4-phenyl-buttersäureethylester, Fp. 68—70°C. Das Filtrat wird am Rationsverdampfer eingeengt, im Hochvakuum bei 45°C entgast und ergibt 146,5 g angereicherten (+)-R-2-(4-Nitrobenzolsulfonyloxy)-4-phenyl-buttersäureethylester vom Drehwert $[\alpha]_D^{20} = 10,6°$ (3%, Ethanol, abs.) Auf diese Weise hergestelltes Produkt hat einen Enantiomerenüberschuss von 90% und gemäss HPLC einen Gehalt von 95%.

Nach obiger Vorschrift aus reinem (−)-R-2-Hydroxy-4-phenyl-buttersäureethylester ($[\alpha]_D^{20} = -20,8°$, 1% Chloroform) hergestellter (+)-R-2-(4-Nitrobenzolsulfonyloxy)-4-phenyl-buttersäureethylester zeigt einen Drehwert von $[\alpha]_D^{20} = +13,2°$ (3% Ethanol abs.).

Beispiel 7

Auf analoge Weise wie in Beispiel 6 wird aus (−)-R-2-Hydroxy-4-phenyl-buttersäureethylester (82% ee) mit 2-Nitrobenzolsulfonylchlorid der entsprechende (−)-R-2-(2-Nitrobenzolsulfonyloxy)-4-phenyl-buttersäureethylester hergestellt. Der mit 95% Ausbeute als Oel anfallende Ester zeigt einen Drehwert won $[\alpha]_D^{20} = -9,6°$ (3% Ethanol abs.).

Beispiel 8

Auf analoge Weise wie in Beispiel 6 wird aus (−)-R-2-Hydroxy-4-phenyl-buttersäureethylester (82% ee) mit 3-Nitrobenzolsulfonylchlorid der (+)-R-2-(3-Nitrobenzolsulfonyloxy)-4-phenyl-buttersäureethylester hergestellt, $[\alpha]_D^{20} = -6,9°$ (3% Ethanol abs.).

Beispiel 9

Auf analoge Weise wie in Beispiel 6 wird aus (−)-R-2-Hydroxy-4-phenyl-buttersäureethylester (84% ee) mit Pentafluorobenzolsulfonylchlorid in 98% Ausbeute (−)-R-2-(Pentafluorbenzolsulfonyloxy)-4-phenyl-buttersäureethylester erhalten, F. 64—65°C (aus Ether/Cyclohexan);
$[\alpha]_D^{20} = -2,5° \pm 0,2°$; $\alpha_{436}^{20} = 12,4°$ (5% Chloroform).

Beispiel 10

Auf analoge Weise wie in Beispiel 6 wird aus (−)-R-2-Hydroxy-4-phenyl-buttersäureethylester (100% ee) mit 2,4-Nitrobenzolsulfonylchlorid in 95% Ausbeute (−)-R-2-(2,4-Dinitrobenzolsulfonyloxy)-4-phenyl-buttersäureethylester als Oel erhalten. Das Oel wird Essigester/Cyclohexan (1:4) kristallisiert und ergibt ein fast weisses Kristallisat (83.7% Ausbeute) Fp. 69—71°C; $[\alpha]_D^{20} = -10,6°$ (3% Ethanol abs.).

Beispiel 11

393,4 g angereicherter (+)-R-2-(4-Nitrobenzolsulfonyloxy)-4-phenyl-buttersäureethylester (90% ee) werden in 600 ml Acetronitril gelöst und bei Raumtemperatur mit 121,4 g Triethylamin versetzt. Nach Aufheizen auf 70°C werden innerhalb von 2 Stunden 210 g (+)-R-1-Phenylethylamin zugetropft. Anschliessend wird weitere 16 Stunden bei 70°C gerührt. Nach vollständigem Umsatz wird abgekühlt, vom ausgefallenen Ammoniumsalz abfiltriert und das Filtrat eingedempft. Dann wird zwischen Wasser und Dichlormethan verteilt, die wässrige Phase mit 2 N Salzsäure auf pH g gestellt. Die vereinigten organischen Phasen werden am Rotationsverdampfer eingeengt, das erhaltene Oel wird anschliessend in einem Gemisch aus 1000 ml Diethylester und 250 ml Dichlormethan gelöst und unter Rühren mit Chlorwasserstoffgas gesättigt. Die ausgefallene Kristallsuspension wird bei 0°C mit 700 ml Cyclohexan versetzt und bei −12°C filtriert, mit Cyclohexan gewaschen und im Hochvakuum bis zur Gewichtskonstanz getrocknet. Die Ausbeute beträgt 287,5 g. Das durch HPLC bestimmte Diastereomerenvenverhältnis beträgt SR:SS = 98,5 : 1,5. Durch einmaliges Umkristallisieren wird N-(R-1-Phenylethyl)-S-homophenyl-alaninethylesterhydrochlorid als reines SR-Isomer erhalten, Fp. 181,5—182,5°C; $[\alpha]_D^{20} = +52,5°$ (1%, Methanol).

Beispiel 12

86,88 g N-(R-1-Phenylethyl-S-homophenylalaninethyl-esterhydrochlorid vom Drehwert +52,5° werden in 870 ml Ethanol und 87 ml deionisiertem Wasser gelöst und mit 17 g Pd/C (5%) bei Normaldruck eind Stunde hydriert. Nach 109% Wasserstoffaufnahme wird die Hydrierung abgebrochen. Nach Filtrieren wird das Filtrat auf ein Volumen von ca. 200 ml aufkonzentriert. Unter Rühren werden 750 ml Diethylether zugetropft, und die enstandene Kristallsuspension wird auf 0°C gekühlt, abfiltriert und mit eiskaltem Ether gewaschen. Nach Trocknen im Hockvakuum erhält man 55,23 g (+)-S-Homophenylalaninethylester-

hydrochlorid vom Drehwert $[\alpha]_D^{20} = +41{,}1°$ (1%, Ethanol). Aus der Mutterlauge werden durch Aufkonzentrieren nachmals 3,96 g Produkt von vergleichbarer Reinheit erhalten.

Beispiel 13

Aufkonzentrierte ethanolische Hydrierlösung (0.7 molarer Ansatz) aus Beispiel 12 wird mit 500 ml Methanol verdünnt und 36 Stunden mit einer Lösung aus 58,8 g Natriumhydroxid und 58,8 Wasser bei Raumtemperatur gerührt. Es wird vom ausgefallenen Natriumchlorid abfiltriert. Das Filtrat wird auf circa 300 ml aufkonzentriert, wobei bereits Homophenylalanin-Natruimsalz auszukristallisieren beginnt. Die Kristallisation wird durch Zutropfen von 1000 ml Acetonitril under Rühren und anschliessendes Abkühlen auf 0°C vervollständigt. Das Produkt wird abfiltriert, mit kaltem Acetronitril gewaschen und bei Raumtemperatur bis zur Gewichtskonstanz im Hochvakuum getrocknet. Das isolierte Natriumsalz hat einen Drehwert von $[\alpha]_D^{20} = +37{,}2°$ (1%, 1 N Salzsäure).

Beispiel 14

10,0 g Homophenylalanin-Natrium werden in 60 ml deionisiertem Wasser gelöst und innerhaln von 1 Stunde zu 24 ml 2 N Salzsäure getropft. Es ensteht wine weisse, leicht glänzende Kristallisation. Diese Suspension Wird mit 1 N Natronlauge auf pH 4,0 eingestellt, dann noch weitere 2 Stunden bei Raumtemperatur gerührt, filtriert und mit deionisiertem Wasser gewaschen. Nach Trocken bei Raumtemperatur im Hochvakuum erhält man reines (+)-S-Homophenylalanin vom Drehwert $[\alpha]_D^{20} = +45{,}6°$ (1%, 1 N Salzsäure), Fp. 287—290°C, Ausbeute: 89,6%.

Beispiel 15

In einem Autoklaven werden 78,7 g (+)-R-2-(4-Nitrobenzolsulfonyloxy)-4-phenyl-buttersäureethylester in 40 ml Acetonitril vergelegt und mit 7.5 g Ammoniak bei 60°C und 12—18 bar zur Reaktion gebracht. Nach 6—7 Stunden ist die Reaktion vollständig. Die Reaktionslösung wird eingedampft, der Rückstand in 200 ml Deithylether aufgenommen. Die Etherlösung wird mit 130 ml Chlorwasserstoff in Essigester (1,7 N) versetzt, worauf nahezu weisser kristalliner (+)-S-Homophenylalaninethylester vom Drehwert $[\alpha]_D^{20} = +37{,}8°$ ausfällt, Ausbeute: 95,6%. Ein Drehwert von + 37,8° (1%, Ethanol) entspricht 93% ee.

Beispiel 16

1-Carboxymethyl-3S-[(1S-ethoxycarbonyl-3-phenylpropyl)amino]-2,3,4,5-tetrahydro-1H-[1]-benzazepin-2-on

46,1 g 1-tert.-Butoxycarbonylether-3S-amino-2,3,4,5-tetrahydro-1H)-[1]-benzazepin-2-on, 84,3 g auf 90% ee angereicherter (+)-R-2-(4-Nitrobenzolsulfonyloxy)-4-phenyl-buttersäureethylester und 19,53 g N-Methylmorpholin werden ohne Lösungsmittel bei 75-80°C 9 Stunden zur Reaktion gebracht. Das ausgefallene N-Methylmorpholinsalz der 4-Nitrobenzolsulfonsäure wird durch Zugabe von 250 ml Essigester und 150 ml Wasser in Lösung gebracht. Mit ca. 150 ml 2 N Sodalösung wird pH 8,8 eingestellt, die Essigesterphase abgetrannt und noch zweimal mit Wasser gewaschen. Das nach Abdestillieren des Essigesters zurückbleibende Oel (98 g) zeigt im HPLC ein Diastereomerenverhältnis von SS:SR = 96:4.

Die Herstellung des Rohwirkstoffes erfolgt duren Einleiten von 54 g Chlorwasserstoffgas in eine Lösung von 96 g obigen Oels in 200 ml Essigester bie 0—10°C. Nach vollständiger Solvolyse des tert.-Butylesters fällt der Rohwirkstoff als feinkristalline Suspension an. Der überschüssige Chlorwasserstoff wird durch wiederholtes Abdestillieren von Essigester im Vakuum vollständig entfernt. Anschliessend wird die start aufkonzentrierte Kristallsuspension mit 200 ml Aceton verdünnt, bei 15°C abfiltriert und mit zweimal je 50 ml Essigester gewaschen. Nach Trocken im Vakuum bei 60°C bis zur Gewichtskonstanzt werden 62,5 g (85.4%) praktisch weisser Wirkstoff mit einem Diastereomerenverhältnis von SS:SR = 99,1 : 0.9 isoliert. Diese 62.5 g Rohwirkstoff werden zur weiteren Reinigung in 250 ml Essigester suspendiert, 6 Stunden unter Rückfluss erhitzt, bei 15°C filtriert, gewaschen und bei 60°C im Hochvakuum getrocknet. Ausbeute: 61,15 g (83.6%), Vehältnis SS:SR = 99,7 : 0,3; $[\alpha]_D^{20} = -137{,}3°$ (1%, Ethanol abs.). Fp. 181°C.

**Patentansprüche für die Vertragsstaaten: BE CH DE FR GB IT LI NL SE**

1. Verbindungen der Formel I,

$$R^1-CH_2-CH_2-\underset{*}{\overset{\overset{\displaystyle COOR^2}{|}}{C}}H-OSO_2-R^3 \qquad (I)$$

worin $R^1$ für unsubstituiertes oder durch $C_1$—$C_7$-Alkyl substituiertes $C_5$—$C_6$-Cycloalkyl, oder für unsubstituiertes oder substituiertes Phenyl steht, $R^2$ $C_1$—$C_7$-Alkyl bedeutet, $R^3$ für Phenyl steht, das durch Halogen oder Nitro substituiertes ist, und das Symbol * ein Kohlenstoffatom kennzeichnet, das entweder bei der überwiegenden Zahl von Molekeln in S- oder bei dur überwiegenden Zahl von Molekeln in R-Konfiguration vorliegt.

2. Verbindungen der Formel I nach Anspruch 1, worin $R^2$, $R^3$ und * die Bedeutungen wie in Anspruch 1 haben und worin $R^1$ für Phenyl steht, das mit einem oder mehreren Substituenten aus der Gruppe $C_1$—$C_7$-Alkyl, Hydroxy, $C_1$—$C_7$-Alkoxy, $C_1$—$C_7$-Alkanoyloxy, Fluor, $C_1$—$C_7$-Alkylendioxy, Amino, $C_1$—$C_7$-Alkylamino, Di-($C_1$—$C_7$)-alkylamino, $C_1$—$C_7$-Alkanoylamino, Carbamoyl, $C_1$—$C_7$-Alkylcarbamoyl, Di-($C_1$—$C_7$)-alkylcarbamoyl, $C_1$—$C_7$-Alkansulfonylamino, Sulfamoyl, $C_1$—$C_7$-Alkylsulfamoyl, Di-($C_1$—$C_7$-)-alkylsulfamoyl, Halogen-$C_1$—$C_7$-alkyl, Hydroxy-$C_1$—$C_7$-alkyl und Amino-$C_1$—$C_7$-alkyl substituiertes sein kann.

3. Verbindungen der Formel I nach Anspruch 1, worin $R^2$, $R^3$ und * die Bedeutungen wie in Anspruch 1 haben und worin $R^1$ für $C_5$—$C_6$-Cycloalkyl, Phenyl oder für durch $C_1$—$C_7$-Alkyl, Hydroxy, $C_1$—$C_7$-Alkoxy, $C_1$—$C_7$-Alkanoyloxy, Fluor, Trifluormethyl oder $C_1$—$C_7$-Alkylendioxy substituiertes Phenyl steht.

4. Verbindungen der Formel I nach Anspruch 1, worin $R^1$ für $C_5$—$C_6$-Cycloalkyl, Phenyl oder durch einen Reste $C_1$—$C_4$-Alkyl. Hydroxy, $C_1$—$C_4$-Alkoxy oder Fluor substituiertes Phenyl steht, $R^2$ $C_1$—$C_4$-Alkyl bedeutet, $R^3$ für 2-, 3- oder 4-Nitrophenyl, 2,4-Dinitrophenyl oder für Pentafluorphenyl steht, und das Symbol * ein Kohlenstoffatom kennzeichnet, das entweder bei der überwiegenden Zahl von Molekeln in S- oder bei der überwiegenden Zahl von Molekeln in R-Konfiguration vorliegt.

5. Verbindungen der Formel I nach Anspruch 1, worin $R^1$ für Cyclohexyl, Phenyl, $C_1$—$C_4$-Alkyphenyl oder $C_1$—$C_4$-Alkoxyphenyl steht, $R^2$ $C_1$—$C_4$-Alkyl bedeutet, $R^3$ für 4-Nitrophenyl oder 2,4-Dinitrophenyl steht und das Symbol * ein Kohlenstoffatom kennzeichnet, das bei der überwiegenden Zahl von Molekeln in R-Konfiguration vorliegt.

6. Verbindungen der Formel I nach Anspruch 1, worin $R^1$ für Phenyl steht, $R^2$ $C_1$—$C_4$-Alkyl bedeutet, $R^3$ für 4-Nitrophenyl oder 2,4-Dinitrophenyl steht und das Symbol * ein Kohlenstoffatom kennzeichnet, das bei der überwiegenden Zahl von Molekeln in R-Konfiguration vorliegt.

7. (+)-R-2-(4-Nitrobenzolsulfonyloxy)-4-phenyl-buttersäureethylester und (−)-R-2-(2,4-Dinitrobenzolsulfonyloxy)-4-phenyl-buttersäureethylester.

8. Verfahren zur Herstellung von Verbindungen der in Anspruch 1 gezeigten Formel I, worin $R^1$, $R^2$, $R^3$ und das Symbol * wie vorstehend definiert sind, dadurch gekennzeichnet, dass man einen α-Hydroxyester der Formel II,

$$R^1\text{-}CH_2\text{-}CH_2\text{-}\overset{\displaystyle COOR^2}{\underset{\displaystyle *}{CH}}\text{-}OH \qquad (II)$$

worin $R^1$ und $R^2$ wie für Formel I definiert sind und * ein Kohlenstoffatom kennzeichnet, das entweder bei der überwiegenden Zahl von Molekeln in S- oder bei der überwiegenden Zahl von Molekeln in R-Konfiguration vorliegt, mit einer den Substituenten —OH in den Rest der Formel —$OSO_2$—$R^3$ überführenden Verbindung umsetzt, worin $R^3$ wie für Formel I definiert ist.

9. Verfahren nach Anspruch 8, dadurch gekennzeichnet, dass man ein $R^3$-Sulfonsäureanhydrid verwendet.

10. Verfahren nach Anspruch 8, dadurch gekennzeichnet, dass man ein $R^3$-Sulfonsäurehalogenid verwendet.

11. Verfahren nach Anspruch 8, dadurch gekennzeichnet, dass man ein 4-Nitrobenzolsulfonsäurehalogenid verwendet.

12. Verfahren nach Anspruch 8, dadurch gekennzeichnet, daß man ein 3-Nitrobenzolsulfonsäurehalogenid verwendet.

13. Verfahren nach Anspruch 8, dadurch gekennzeichnet, dass man ein 2,4-Dinitrobenzolsulfonsäurehalogenid verwendet.

14. Verfahren nach Anspruch 8, dadurch gekennzeichnet, dass man ein Pentafluorbenzolsulfonsäurehalogenid verwendet.

15. Verfahren nach Anspruch 8, dadurch gekennzeichnet, dass man ein Anhydrid vom Typus $R^3$—$SO_2$—O—$SO_2$—$R^3$ verwendet.

16. Verfahren zur Herstellung von Verbindungen der in Anspruch 1 gezeigten Formal I, worin $R^1$, $R^2$, $R^3$ und das Symbol * wie vorstehend definiert sind, dadurch gekennzeichnet, dass man eine Verbindung der Formel III,

$$R^1\text{-}CH_2\text{-}CH_2\text{-}\overset{\displaystyle COOR^2}{C}{=}O \qquad (III)$$

worin $R^1$ und $R^2$ wie für Formel I definiert sind, in Gegenwart eines Platinkatalysators auf einem Träger

sowie eines Cinchonaalkaloides zu einer Verbindung der Formel II, die wie vorstehend definiert ist, enantioselektiv reduziert,

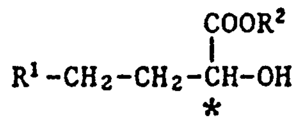

(II)

und die erhaltene Verbindung der Formel II mit einer den Substituenten —OH in den Rest der Formel $—OSO_2—R^3$ überführenden Verbindung umsetzt, worin $R^3$ wie für Formel I definiert ist.

17. Verfahren nach Anspruch 16, dadurch gekennzeichnet, dass es sich bei dem Träger um Kohle, Aluminiumoxid, Calciumcarbonat oder Bariumsulfat handelt.

18. Verfahren nach Anspruch 16, dadurch gekennzeichnet, dass es sich bei dem Cinchonaalkaloid um (−)-Chinin, (+)-Chinidin, (+)-Cinchonin oder (−)-Cinchonidin handelt.

19. Verfahren nach Anspruch 16, dadurch gekennzeichnet, dass es sich bei dem Cinchonaalkaloid um (−)-Cinchonidin handelt.

20. Verfahren nach Anspruch 16, dadurch gekennzeichnet, dass mann von 4-Phenyl-2-oxo-buttersäureethylester ausgeht.

21. Verwendung von Verbindungen der in Anspruch 1 gezeigten Formel I zur Herstellung von Verbindungen der Formel VI,

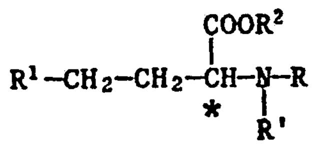

(VI)

dadurch gekennzeichnet, dass man eine Verbindung der Formel VII

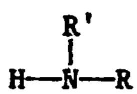

(VII)

unter Inversion mit einer Verbindung der Formel I

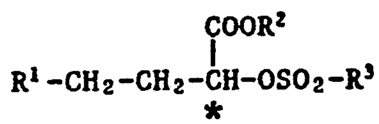

(I)

alkyliert, wobei $R^1$, $R^2$ und $R^3$ wie vorstehend definiert sind, das Symbol * ein Kohlenstoffatom kennzeichnet, das entweder bei der überwiegenden Zahl von Molekeln in S- oder bei der überwiegenden Zahl von Molekeln in R-Konfiguration vorliegt, und wobei R′ Wasserstoff oder $C_1—C_7$-Alkyl ist und R den Rest der Teilformel VIII

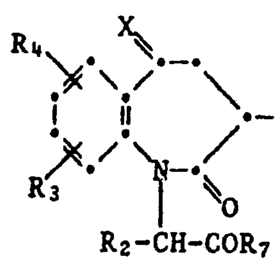

(VIII)

bedeutet, wobei in dieser Teilformel VIII die Reste $R_2$, $R_3$, $R_4$, $R_7$ und X die Bedeutungen haben, die in der

europäischen Patentanmeldung 72 352 definiert sind, oder wobei R' Wasserstoff oder $C_1$—$C_7$-Alkyl ist und R den Rest der Teilformel IX

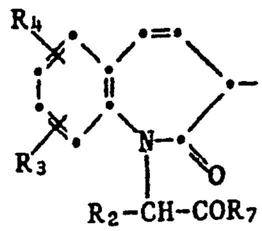

(IX)

bedeutet, wobei in dieser Teilformel IX die Reste $R_2$, $R_3$, $R_4$, $R_7$ die Bedeutungen haben, die in der europäischen Patentanmeldung 72 352 definiert sind, oder wobei R' Wasserstoff und R für 1-Aryl-$C_1$—$C_7$-alkyl steht, oder wobei R' und R Wasserstoff bedeuten.

22. Verwendung von Verbindungen der in Anspruch 1 gezeigten Formel I nach Anspruch 21 zur Herstellung von Verbindungen der Formel VIa,

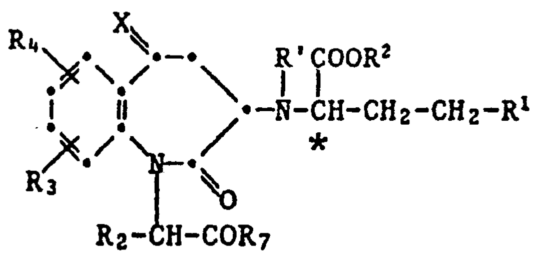

(VIa)

worin $R^1$, $R^2$, R', * sowie $R_2$, $R_3$, $R_4$, $R_7$ und X wie in Anspruch 21 definiert sind.

23. Verwendung von Verbindungen der in Anspruch 1 gezeigten Formel I nach Anspruch 21 zur Herstellung von Verbindungen der Formel VIb

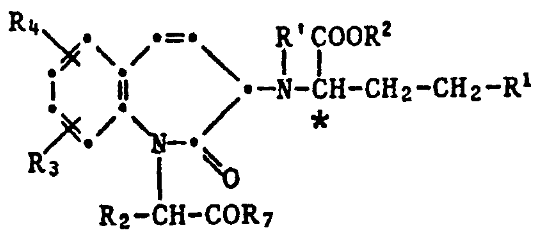

(VIb)

worin $R^1$, $R^2$, R', * sowie $R_2$, $R_3$, $R_4$, $R_7$ wie in Anspruch 21 definiert sind.

24. Verwendung von Verbindungen der in Anspruch 23 gezeigten Formel VIb zur Herstellung von Verbindungen der in Anspruch 22 gezeigten Formel VIa, worin X zwei Wasserstoffatome darstellt, dadurch gekennzeichnet, dass man eine Verbindung der Formel VIb hydrogenolysiert.

25. Verwendung von Verbindungen der in Anspruch 1 gezeigten Formel I zur Anspruch 21 zur Herstellung von Verbindungen der Formel VIc,

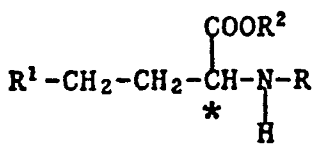

(VIc)

worin $R^1$, $R^2$ und * wie vorstehend definiert sind und R für 1-Aryl-$C_1$—$C_7$-alkyl, z.B. für Benzyl, 1-Phenylethyl oder 1-Naphthylethyl, steht.

26. Verwendung von Verbindungen der in Anspruch 1 gezeigten Formel I nach Anspruch 21 zur Herstellung von Verbindungen der Formel VId

$$R^1-CH_2-CH_2-\overset{\overset{\displaystyle COOR^2}{|}}{\underset{*}{C}}H-NH_2 \qquad (VId)$$

worin $R^1$, $R^2$ und * wie vorstehend definiert sind.

27. Verwendung von Verbindungen der in Anspruch 1 gezeigten Formel I nach Anspruch 21 zur Herstellung von 1-Carboxymethyl-3S-[(1S-ethoxycarbonyl-3-phenyl-propyl)amino]-2,3,4,5-tetrahydro-1H-[1]-benzazepin-2-on.

28. (+)-R-2-(3-Nitrobenzolsulfonyloxy)-4-phenyl-buttersäureethylester.

**Patentansprüche für den Vertragsstaat: AT**

1. Verfahren zur Herstellung von Verbindungen der Formel I,

$$R^1-CH_2-CH_2-\overset{\overset{\displaystyle COOR^2}{|}}{\underset{*}{C}}H-OSO_2-R^3 \qquad (I)$$

worin $R^1$ für unsubstituiertes oder durch $C_1$—$C_7$-Alkyl substituiertes $C_5$—$C_6$-Cycloalkyl, oder für unsubstituiertes oder substituiertes Phenyl steht, $R^2$ $C_1$—$C_7$-Alkyl bedeutet, $R^3$ für Phenyl steht, das durch Halogen oder Nitro substituiert ist, und das Symbol * ein Kohlenstoffatom kennzeichnet, das entweder bei der überwiegenden Zahl von Molekeln in S- oder bei dur überwiegenden Zahl von Molekeln in R-Konfiguration vorliegt, dadurch gekennzeichnet, dass man einen α-Hydroxyester der Formel II,

$$R^1-CH_2-CH_2-\overset{\overset{\displaystyle COOR^2}{|}}{\underset{*}{C}}H-OH \qquad (II)$$

worin $R^1$ und $R^2$ wie für Formel I definiert sind und * ein Kohlenstoffatom kennzeichnet, das entweder bei der überwiegenden Zahl von Molekeln in S- oder bei der überwiegenden Zahl on Molekeln in R-Konfiguration vorliegt, mit einer den Substituenten —OH in den Rest der Formel —$OSO_2$—$R^3$ überführenden Verbindung umsetzt, worin $R^3$ wie für Formel I definiert ist,

2. Verfahren nach Anspruch 1 zur Herstellung von Verbindungen der Formel I, worin $R^2$, $R^3$ und * die Bedeutungen wie in Anspruch 1 haben und worin $R^1$ für Phenyl steht, das mit einem oder mehreren Substituenten aus der Gruppe $C_1$—$C_7$-Alkyl, Hydroxy, $C_1$—$C_7$-Alkoxy, $C_1$—$C_7$-Alkanoyloxy, Fluor, $C_1$—$C_7$-Alkylendioxy, Amino, $C_1$—$C_7$-Alkylamino, Di-($C_1$—$C_7$)-alkylamino, $C_1$—$C_7$-Alkanoylamino, Carbamoyl, $C_1$—$C_7$-Alkylcarbamoyl, Di-($C_1$—$C_7$)-alkylcarbamoyl, $C_1$—$C_7$-Alkansulfonylamino, Sulfamoyl, $C_1$—$C_7$-Alkylsulfamoyl, Di-($C_1$—$C_7$-)-alkylsulfamoyl, Halogen-$C_1$—$C_7$-alkyl, Hydroxy-$C_1$—$C_7$-alkyl und Amino-$C_1$—$C_7$-alkyl substituierte sein kann.

3. Verfahren nach Anspruch 1 zur Herstellung von Verbindungen der Formel I, worin $R^2$, $R^3$ und * die Bedeutungen wie in Anspruch 1 haben und worin $R^1$ für $C_5$—$C_9$-Cycloalkyl, Phenyl oder für durch $C_1$—$C_7$-Alkyl, Hydroxy, $C_1$—$C_7$-Alkoxy, $C_1$—$C_7$-Alkanoyloxy, Fluor, Trifluormethyl oder $C_1$—$C_7$-Alkylendioxy substituiertes Phenyl steht.

4. Verfahren nach Anspruch 1 zur Herstellung von Verbindungen der Formel I, worin $R^1$ für $C_5$—$C_6$-Cycloalkyl, Phenyl oder durch einen Reste $C_1$—$C_4$-Alkyl, Hydroxy, $C_1$—$C_4$-Alkoxy oder Fluor substituiertes Phenyl steht, $R^2$ $C_1$—$C_4$-Alkyl bedeutet, $R^3$ für 2-, 3- oder 4-Nitrophenyl, 2,4-Dinitrophenyl oder für Pentafluorphenyl steht, und das Symbol * ein Kohlenstoffatom kennzeichnet, das entweder bei der überwiegenden Zahl von Molekeln in S- oder bei der überwiegenden Zahl von Molekeln in R-Konfiguration vorliegt.

5. Verfahren nach Anspruch 1 zur Herstellung von Verbindungen der Formel I, worin $R^1$ für Cyclohexyl, Phenyl, $C_1$—$C_4$-Alkyphenyl oder $C_1$—$C_4$-Alkoxyphenyl steht, $R^2$ $C_1$—$C_4$-Alkyl bedeutet, $R^3$ für 4-Nitrophenyl oder 2,4-Dinitrophenyl steht und das Symbol * ein Kohlenstoffatom kennzeichnet, das bei der überwiegenden Zahl von Molekeln in R-Konfiguration vorliegt.

6. Verfahren nach Anspruch 1 zur Herstellung von Verbindungen der Formel I, worin $R^1$ für Phenyl steht, $R^2$ $C_1$—$C_4$-Alkyl bedeutet, $R^3$ für 4-Nitrophenyl oder 2,4-Dinitrophenyl steht und das Symbol * ein Kohlenstoffatom kennzeichnet, das bei der überwiegenden Zahl von Molekeln in R-Konfiguration vorliegt.

7. Verfahren nach Anspruch 1 zur Herstellung von (+)-R-2-(4-Nitrobenzolsulfonyloxy)-4-phenyl-buttersäureethylester oder (−)-R-2-(2,4-Dinitrobenzolsulfonyloxy)-4-phenyl-buttersäureethylester.

8. Verfahren nach Anspruch 1 zur Herstellung von (+)-R-2-(3-Nitrobenzolsulfonyloxy)-4-phenyl-buttersäureethylester.

9. Verfahren nach Anspruch 1, dadurch gekennzeichnet, dass man ein $R^3$-Sulfonsäureanhydrid verwendet.

10. Verfahren nach Anspruch 1, dadurch gekennzeichnet, dass man ein $R^3$-Sulfonsäurehalogenid verwendet.

11. Verfahren nach Anspruch 1, dadurch gekennzeichnet, dass man ein 4-Nitrobenzolsulfonsäurehalogenid verwendet.

12. Verfahren nach Anspruch 1, dadurch gekennzeichnet, dass man ein 3-Nitrobenzolsulfonsäurehalogenid verwendet.

13. Verfahren nach Anspruch 1, dadurch gekennzeichnet, dass man ein 2,4-Dinitrobenzolsulfonsäurehalogenid verwendet.

14. Verfahren nach Anspruch 1, dadurch gekennzeichnet, dass man ein Pentafluorbenzolsulfonsäurehalogenid verwendet.

15. Verfahren nach Anspruch 1, dadurch gekennzeichnet, dass man ein Anhydrid vom Typus $R^3—SO_2—O—SO_2—R^3$ verwendet.

16. Verfahren zur Herstellung von Verbindungen der in Anspruch 1 gezeigten Formal I, worin $R^1$, $R^2$, $R^3$ und das Symbol * wie vorstehend definiert sind, dadurch gekennzeichnet, dass man eine Verbindung der Formel III,

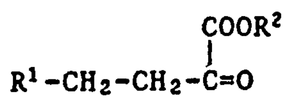

(III)

worin $R^1$ und $R^2$ wie für Formel I definiert sind, in Gegenwart eines Platinkatalysators auf einem Träger sowie eines Cinchonaalkaloides zu einer Verbindung der Formel II, die wie vorstehend definiert ist, enantioselektiv reduziert,

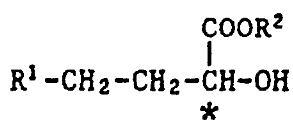

(II)

und die erhaltene Verbindung der Formel II mit einer den Substituenten —OH in den Rest der Formel $—OSO_2—R^3$ überführenden Verbindung umsetzt, worin $R^3$ wie für Formel I definiert ist.

17. Verfahren nach Anspruch 16, dadurch gekennzeichnet, dass es sich bei dem Träger um Kohle, Aluminiumoxid, Calciumcarbonat oder Bariumsulfat handelt.

18. Verfahren nach Anspruch 16, dadurch gekennzeichnet, dass es sich bei dem Cinchonaalkaloid um (−)-Chinin, (+)-Chinidin, (+)-Cinchonin oder (−)-Cinchonidin handelt.

19. Verfahren nach Anspruch 16, dadurch gekennzeichnet, dass es sich bei dem Cinchonaalkaloid um (−)-Cinchonidin handelt.

20. Verfahren nach Anspruch 16, dadurch gekennzeichnet, dass man von 4-Phenyl-2-oxo-buttersäureethylester ausgeht.

21. Verwendung von Verbindungen der in Anspruch 1 gezeigten Formel I zur Herstellung von Verbindungen der Formel VI,

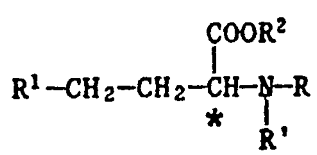

(VI)

dadurch gekennzeichnet, dass man eine Verbindung der Formel VII

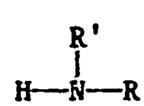

(VII)

unter Inversion mit einer Verbindung der Formel I

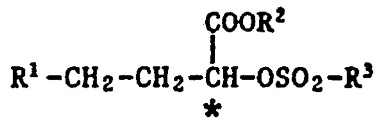

(I)

alkyliert, wobei $R^1$, $R^2$ und $R^3$ wie vorstehend definiert sind, das Symbol * ein Kohlenstoffatom kennzeichnet, das entweder bei der überwiegenden Zahl von Molekeln in S- oder bei der überwiegenden Zahl von Molekeln in R-Konfiguration vorliegt, und wobei R' Wasserstoff oder $C_1$—$C_7$-Alkyl ist und R den Rest der Teilformel VIII

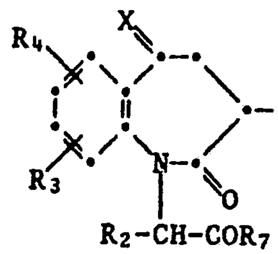

(VIII)

bedeutet, wobei in dieser Teilformel VIII die Reste $R_2$, $R_3$, $R_4$, $R_7$ und X die Bedeutungen haben, die in der europäischen Patentanmeldung 72 352 definiert sind, oder wobei R' Wasserstoff oder $C_1$—$C_7$-Alkyl ist und R den Rest der Teilformel IX

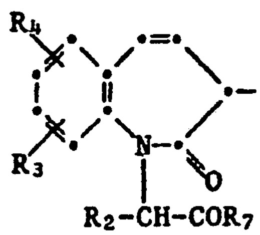

(IX)

bedeutet, wobei in dieser Teilformel IX die Reste $R_2$, $R_3$, $R_4$, $R_7$ die Bedeutungen haben, die in der europäischen Patentanmeldung 72 352 definiert sind, oder wobei R' Wasserstoff und R für 1-Aryl-$C_1$—$C_7$-alkyl steht, oder wobei R' und R Wasserstoff bedeuten.

22. Verwendung von Verbindungen der in Anspruch 1 gezeigten Formel I nach Anspruch 21 zur Herstellung von Verbindungen der Formel VIa,

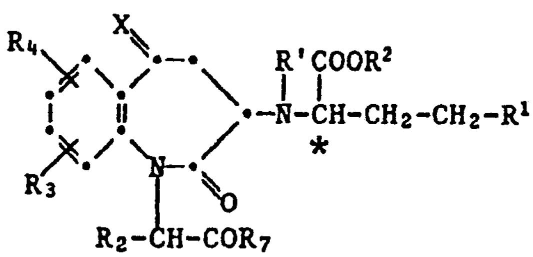

(VIa)

worin $R^1$, $R^2$, R', * sowie $R_2$, $R_3$, $R_4$, $R_7$ und X wie in Anspruch 21 definiert sind.

23. Verwendung von Verbindungen der in Anspruch 1 gezeigten Formel I nach Anspruch 21 zur Herstellung von Verbindungen der Formel VIb

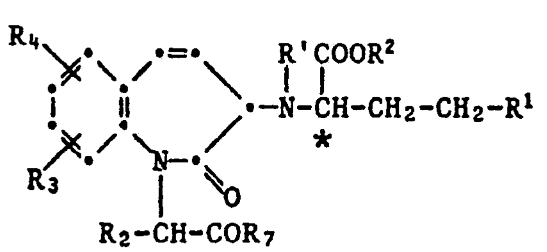

(VIb)

worin $R^1$, $R^2$, R', * sowie $R_2$, $R_3$, $R_4$, $R_7$ wie in Anspruch 21 definiert sind.

24. Verwendung von Verbindungen der in Anspruch 23 gezeigten Formel VIb zur Herstellung von Verbindungen der in Anspruch 22 gezeigten Formel VIa, worin X zwei Wasserstoffatome darstellt, dadurch gekennzeichnet, dass man eine Verbindung der Formel VIb hydrogenolysiert.

25. Verwendung von Verbindungen der in Anspruch 1 gezeigten Formel I zur Anspruch 21 zur Herstellung von Verbindungen der Formel VIc,

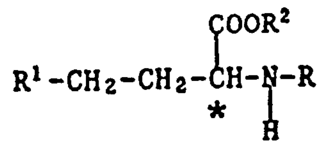

(VIc)

worin $R^1$, $R^2$ und * wie vorstehend definiert sind und R für 1-Aryl-$C_1$—$C_7$-alkyl, z.B. für Benzyl, 1-Phenylethyl oder 1-Naphthylethyl, steht.

26. Verwendung von Verbindungen der in Anspruch 1 gezeigten Formel I nach Anspruch 21 zur Herstellung von Verbindungen der Formel VId

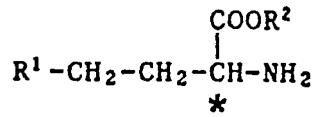

(VId)

worin $R^1$, $R^2$ und * wie vorstehend definiert sind.

27. Verwendung von Verbindungen der in Anspruch 1 gezeigten Formel I nach Anspruch 21 zur Herstellung von 1-Carboxymethyl-3S-[(1S-ethoxycarbonyl-3-phenyl-propyl)amino]-2,3,4,5-tetrahydro-1H-[1]-benzazepin-2-on.

**Revendications pour les Etats contractants: BE CH DE FR GB IT LI NL**

1. Composés de formule I:

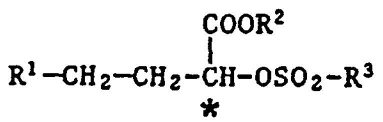

(I)

où $R^1$ représente un cycloalkyle en $C_5$—$C_6$ non substitué ou substitué par un alkyle en $C_1$—$C_7$ ou le phényle non substitué ou substitué, $R^2$ représente un alkyle en $C_1$—$C_7$, $R^3$ représente le phényle substitué par un halogène ou le nitro, et le symbole * marque un atome de carbone se présentant, soit dans un nombre prédominant de molécules dans la configuration S, soit dans un nombre prédominant de molécules dans la configuration R.

2. Composés de formule I selon la revendication 1, où $R^2$, $R^3$ et * ont la même signification que dans la revendication 1 et où $R^1$ représente le phényle pouvant être substitué par un ou plusieurs substituants provenant du groupe constitué par un alkyle en $C_1$—$C_7$, l'hydroxy, un alcoxy en $C_1$—$C_7$, un alcanoyl en $C_1$—$C_7$ oxy, le fluor, un alkylene en $C_1$—$C_7$ dioxy, l'amino, un alkyle en $C_1$—$C_7$ amino, un dialkyle en $C_1$—$C_7$ amino, un alcanoyl en $C_1$—$C_7$ amino, un carbamoyl, un alkyle en $C_1$—$C_7$ carbamoyl, un dialkyle en $C_1$—$C_7$ carbamoyl, un alcane en $C_1$—$C_7$ sulfonylamino, le sulfamoyl, un alkyle en $C_1$—$C_7$ sulfamoyl, un dialkyle en $C_1$—$C_7$ sulfamoyl, un halogénoalkyle en $C_1$—$C_7$, un hydroxyalkyle en $C_1$—$C_7$ et un aminoalkyle en $C_1$—$C_7$.

3. Composés de formule I selon la revendication 1 où $R^2$, $R^3$ et * ont la mêmes signification que dans la revendication 1 et où $R^1$ représente un cycloalkyle en $C_5$—$C_6$, le phényle ou le phényle substitué par un alkyle en $C_1$—$C_7$, l'hydroxy, un alcoxy en $C_1$—$C_7$, un alcanoyl en $C_1$—$C_7$ oxy, le fluor, le trifluorométhyle ou le phényle substitué par un alkylène en $C_1$—$C_7$ dioxy.

4. Composés de formule I selon la revendication 1 où $R^1$ représente un cycloalkyle en $C_5$—$C_6$, le phényle ou le phényle substitué par l'un des restes alkyle en $C_1$—$C_4$, l'hydroxy, un alcoxy en $C_1$—$C_4$ ou le fluor, $R^2$ représente un alkyle en $C_1$—$C_4$, $R^3$ représente le 2-, le 3- ou le 4-nitrophényle, le 2,4-dinitrophényle ou le pentafluorophényle, et le symbole * marque un atome de carbone se présentant, soit dans un nombre prédominant de molécules dans la configuration S, soit dans un nombre prédominant de molécules dans la configuration R.

5. Composés de formule I selon la revendication 1 où $R^1$ représente le cyclohexyle, le phényle, un alkyle en $C_1$—$C_4$ phényle ou un alcoxy en $C_1$—$C_4$, $R^3$ représente le 4-nitrophényle ou le 2,4-dinitrophényle et le symbole * marque un atome de carbone se présentant dans un nombre prédominant de molécules dans la configuration R.

6. Composés de formule I selon la revendication 1 où $R^1$ représente le phényle, $R^2$ représente un alkyle en $C_1$—$C_4$, $R^3$ représente le 4-nitrophényle ou le 2,4-dinitrophényle et le symbole * marque un atome de carbone se présentant dans un nombre prédominant de molécules dans la configuration R.

7. L'ester éthylique de l'acide (+)-R-2-(4-nitrobenzènesulfonyl)-4-phénylbutyrique et l'ester éthylique de l'acide (−)-R-2-(2,4-dinitrobenzènesulfonyloxy)-4-phénylbutyrique.

8. Procédé de préparation des composés de formule I représentée dans la revendication 1 où $R^1$, $R^2$, $R^3$ et le symbole * sont définis comme précédemment, caractérisé en ce que l'on fait réagir un α-hydroxyester de formule II:

$$R^1-CH_2-CH_2-\underset{*}{\overset{\overset{\displaystyle COOR^2}{|}}{C}}H-OH \qquad (II)$$

où $R^1$ et $R^2$ sont définis comme pour la formule I et * marque un atome de carbone se présentant, soit dans un nombre prédominant de molécules dans la configuration S, soit dans un nombre prédominant de molécules dans la configuration R, avec un composé transformant les substituants OH en un reste de formule $—OSO_2—R^3$ où $R^3$ est défini comme pour la formule I.

9. Procédé selon la revendication 8, caractérisé en ce que l'on utilise un anhydride sulfonique $R^3$.

10. Procédé selon la revendication 8, caractérisé en ce que l'on utilise un halogénure d'acide sulfonique $R^3$.

11. Procédé selon la revendication 8, caractérisé en ce que l'on utilise un halogénure d'acide 4-nitrobenzènesulfonique.

12. Procédé selon la revendication 8, caractérisé en ce que l'on utilise un halogénure d'acide 3-nitrobenzènesulfonique.

13. Procédé selon la revendication 8, caractérisé en ce que l'on utilise un halogénure d'acide 2,4-dinitrobenzènesulfonique.

14. Procédé selon la revendication 8, caractérisé en ce que l'on utilise un halogénure d'acide pentafluorobenzènesulfonique.

15. Procédé selon la revendication 8, caractérisé en ce que l'on utilise un anhydride du type $R^3—SO_2—O—SO_2—R^3$.

16. Procédé pour la préparation des composés de formule I représentée dans la revendication 1 où $R^1$, $R^2$, $R^3$ et le symbole * sont définis comme précédemment, caractérisé en ce que l'on réduit énantiosélectivement un composé de formule III:

$$R^1-CH_2-CH_2-\overset{\overset{\displaystyle COOR^2}{|}}{C}=O \qquad (III)$$

où $R^1$, et $R^2$ sont définis comme pour la formule I, en présence d'un catalyseur à base de platine sur un support ainsi que d'un alcaloïde du cinchona, en un composé de formule II défini comme précédemment:

$$R^1-CH_2-CH_2-\underset{*}{\overset{\overset{\displaystyle COOR^2}{|}}{C}}H-OH \qquad (II)$$

et en ce que l'on fait réagir le composé de formule II obtenu avec un composé transformant les substituants OH en un reste de formule $—OSO_2—R^3$ où $R^3$ est défini comme pour la formule I.

17. Procédé selon la revendication 16, caractérisé en ce que le support est constitué par du charbon, de l'oxyde d'aluminium, du carbonate de calcium ou du sulfate de baryum.

18. Procédé selon la revendication 16, caractérisé en ce que l'alcaloïde du cinchona est la (−)-quinine, la (+)-quinidine, la (+)-cinchonine ou la (−)-cinchonidine.

19. Procédé selon la revendication 16, caractérisé en ce que l'alcaloïde du cinchona est la (−)-conchonidine.

20. Procédé selon la revendication 16, caractérisé en ce que l'on part de l'ester éthylique de l'acide 4-phényl-2-oxobutyrique.

21. Utilisation des composés de formule I représentée dans la revendication 1 pour la préparation des composés de formule VI:

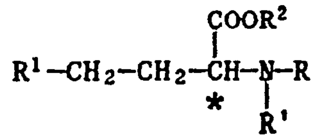

(VI)

caractérisé en ce que l'on alcoyle un composé de formule VII

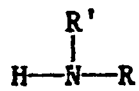

(VII)

sous inversion avec un composé de formule I:

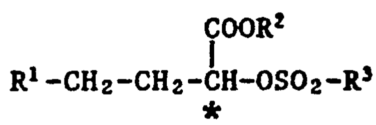

(I)

où $R^1$, $R^2$ et $R^3$ sont définis comme précédemment, le symbole * marque un atome de carbone se présentant, soit dans un nombre prédominant de molécules dans la configuration R, soit dans un nombre prédominant de molécules dans la configuration R et où R' est l'hydrogène ou un alkyle en $C_1$—$C_7$ et R représente le reste de formule partielle VIII

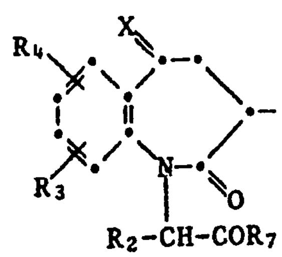

(VIII)

où dans cette formule partielle VIII les restes $R_2$, $R_3$, $R_4$, $R_7$ ont les significations définies dans la demande de brevet européen 72 352 où R' est l'hydrogène ou un alkyle en $C_1$—$C_7$ et R représente le reste de formule partielle IX:

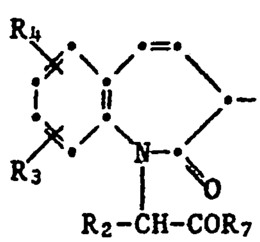

(IX)

où dans cette formule partielle IX les restes $R_2$, $R_3$, $R_4$ et $R_7$ ont les significations définies dans la demande de brevet européen 72 352 où R' représente l'hydrogène et R représente un 1-arylalkyle en $C_1$—$C_7$ où R' et R représentent l'hydrogène.

22. Utilisation des composés de formule I représentée dans la revendication 1 selon la revendication 21 pour la préparation des composés de formule VIa:

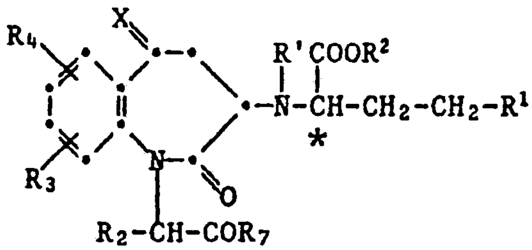

(VIa)

où $R^1$, $R^2$, R', * ainsi que $R_2$, $R_3$, $R_4$, $R_7$ et X sont définis comme dans la revendication 21.

23. Utilisation des composés de formule I représentée dans la revendication 1 selon la revendication 21 pour la préparation des composés de formule VIb:

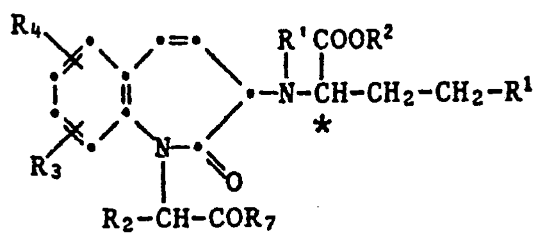

(VIb)

où $R^1$, $R^2$, R', * ainsi que $R_2$, $R_3$, $R_4$ et $R_7$ sont définis comme dans la revendication 21.

24. Utilisation des composés de formule VIb représentée dans la revendication 23 pour la préparation des composés de formule VIa représentée dans la revendication 22 où X représente deux atomes d'hydrogène, caractérisé en ce que l'on hydrogénolyse un composé de formule VIb.

25. Utilisation des composés de formule I représentée dans la revendication 1 selon la revendication 21 pour la préparation des composés de formule VIc:

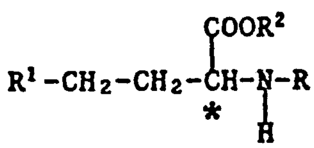

(VIc)

où $R^1$, $R^2$ et * sont définis comme précédemment et où R représente un 1-arylalkyle en $C_1$—$C_7$, par exemple le benzyle, le 1-phényléthyle ou le 1-naphtyléthyle.

26. Utilisation des composés de formule I représentée dans la revendication 1 selon la revendication 21 pour la préparation des composés de formule VId:

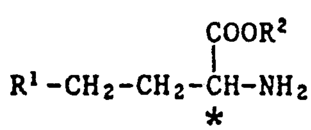

(VId)

où $R^1$, $R^2$ et * sont définis comme précédemment.

27. Utilisation des composés de formule I représentée dans la revendication 1 selon la revendication 21 pour la préparation de la 1-carboxyméthyle-3S-[(1S-éthoxycarbonyl-3-phénylpropyl)-amino]-2,3,4,5-tétrahydro-1H-[1]-benzazépin-2-one.

28. Ester éthylique de l'acide (+)-R-2-(3-nitrobenzènesulfonyloxy)-4-phénylbutyrique.

**Revendications pour l'Etat contractant: AT**

1. Procédé de préparation des composés de formule I

$$R^1-CH_2-CH_2-\underset{*}{\overset{\overset{\displaystyle COOR^2}{|}}{C}}H-OSO_2-R^3 \qquad (I)$$

où $R^1$ représente un cycloalkyle en $C_5$—$C_6$ non substitué ou substitué par un alkyle en $C_1$—$C_7$ ou le phényle non substitué ou substitué, $R^2$ représente un alkyle en $C_1$—$C_7$, $R^3$ représente le phényle substitué par un halogène ou le nitro, et le symbole * marque un atome de carbone se présentant, soit dans un nombre prédominant de molécule dans la configuration S, soit dans un nombre prédominant de molécule dans la configuration R, caractérisé en ce que l'on fait réagir un α-hydroxyester de formule II

$$R^1-CH_2-CH_2-\underset{*}{\overset{\overset{\displaystyle COOR^2}{|}}{C}}H-OH \qquad (II)$$

où $R^1$ et $R^2$ sont définis comme pour la formule I et * marque un atome de carbone se présentant, soit dans un nombre prédominant de molécules dans la configuration S, soit dans un nombre prédominant de molécules dans la configuration R, avec un composé transformant les substituants OH en un reste de formule —$OSO_2$—$R^3$ où $R^3$ est défini comme pour la formule I.

2. Procédé selon la revendication 1, pour la préparation de composés de formule I selon la revendication 1 où $R^2$, $R^3$ et * ont la même signification que dans la revendication 1 et où $R^1$ représente le phényle pouvant être substitué par un ou plusieurs substituants provenant du groupe constitué par un alkyle en $C_1$—$C_7$, l'hydroxy, un alcoxy en $C_1$—$C_7$, un alcanoyl en $C_1$—$C_7$ oxy, le fluor, un alkylene en $C_1$—$C_7$ dioxy, l'amino, un alkyle en $C_1$—$C_7$ amino, un dialkyle en $C_1$—$C_7$ amino, un alcanoyl en $C_1$—$C_7$ amino, un carbamoyl, un alkyle en $C_1$—$C_7$ carbamoyl, un dialkyle en $C_1$—$C_7$ carbamoyl, un alcane en $C_1$—$C_7$ sulfonylamino, le sulfamoyl, un alkyle en $C_1$—$C_7$ sulfamoyl, un dialkyle en $C_1$—$C_7$ sulfamoyl, un halogénoalkyle en $C_1$—$C_7$, un hydroxyalkyle en $C_1$—$C_7$ et un aminoalkyle en $C_1$—$C_7$.

3. Procédé selon la revendication 1, pour la préparation de composés de formule I selon la revendication 1 où $R^2$, $R^3$ et * ont les mêmes significations que dans la revendication 1 et où $R^1$ représente un cycloalkyle en $C_5$—$C_6$, le phényle ou le phényle substitué par un alkyle en $C_1$—$C_7$, l'hydroxy, un alcoxy en $C_1$—$C_7$, un alcanoyl en $C_1$—$C_7$ oxy, le fluor, le trifluorométhyle ou le phényle substitué par un alkylène en $C_1$—$C_7$ dioxy.

4. Procédé selon la revendication 1, pour la préparation de composés de formule I selon la revendication 1 où $R^1$ représente un cycloalkyle en $C_5$—$C_6$, le phényle ou le phényle substitué par l'un des restes alkyle en $C_1$—$C_4$, l'hydroxy, un alcoxy en $C_1$—$C_4$ ou le fluor, $R^2$ représente un alkyle en $C_1$—$C_4$, $R^3$ représente le 2-, le 3- ou le 4-nitrophényle, le 2,4-dinitrophényle ou le pentafluorophényle, et le symbole * marque un atome de carbone se présentant, soit dans un nombre prédominant de molécules dans la configuration S, soit dans un nombre prédominant de molécules dans la configuration R.

5. Procédé selon la revendication 1, pour la préparation de composés de formule I selon la revendication 1 où $R^1$ représente le cyclohexyle, le phényle, un alkyle en $C_1$—$C_4$ phényle ou un alcoxy en $C_1$—$C_4$ phényle, $R^2$ représente un alkyle en $C_1$—$C_4$, $R^3$ représente le 4-nitrophényle ou le 2,4-dinitrophényle et le symbole * marque un atome de carbone se présentant dans un nombre prédominant de molécules dans la configuration R.

6. Procédé selon la revendication 1, pour la préparation de composés de formule I selon la revendication 1, où $R^1$ représente le phényle, $R^2$ représente un alkyle en $C_1$—$C_4$, $R^3$ représente le 4-nitrophényle ou le 2,4-dinitrophényle et le symbole * marque un atome de carbone se présentant dans un nombre prédominant de molécules dans la configuration R.

7. Procédé selon la revendication 1, pour la préparation de l'ester éthylique de l'acide (+)-R-2-(4-nitrobenzènesulfonyl)-4-phényl-butyrique et l'ester éthylique de l'acide (−)-R-2-(2,4-dinitrobenzènesulfonyloxy)-4-phényl-butyrique.

8. Procédé selon la revendication 1, pour la preparation de l'ester éthylique de l'acide (+)-R-2-(3-nitrobenzènesulfonyloxy)-4-phényl-butyrique.

9. Procédé selon la revendication 1, caractérisé en ce que l'on utilise un anhydride sulfonique $R^3$.

10. Procédé selon la revendication 1, caractérisé en ce que l'on utilise un halogénure d'acide sulfonique $R^3$.

11. Procédé selon la revendication 1, caractérisé en ce que l'on utilise un halogénure d'acide 4-nitrobenzènesulfonique.

12. Procédé selon la revendication 1, caractérisé en ce que l'on utilise un halogénure d'acide 3-nitrobenzènesulfonique.

13. Procédé selon la revendication 1, caractérisé en ce que l'on utilise un halogénure d'acide 2,4-dinitrobenzènesulfonique.

14. Procédé selon la revendication 1, caractérisé en ce que l'on utilise un halogénure d'acide pentafluorobenzènesulfonique.

15. Procédé selon la revendication 1, caractérisé en ce que l'on utilise un anhydride du type $R^3—SO_2—O—SO_2—R^3$.

16. Procédé pour la préparation des composés de formule I représentée dans la revendication 1 où $R^1$, $R^2$, $R^3$ et le symbole * sont définis comme précédemment, caractérisé en ce que l'on réduit énantiosélectivement un composé de formule III

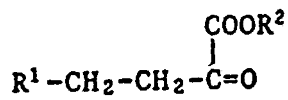

(III)

où $R^1$ et $R^2$ sont définis come pour la formule I, en présence d'un catalyseur à base de platine sur un support ainsi que d'un alcaloïde du cinchona, en un composé de formule II défini comme précédemment

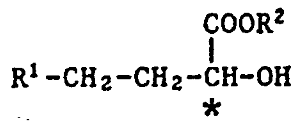

(II)

et en ce que l'on fait réagir le composé de formule II obtenu avec un composé transformant les substituants OH en un reste de formule $—OSO_2—R^3$ où $R^3$ est défini comme pour la formule I.

17. Procédé selon la revendication 16, caractérisé en ce que le support est constitué par du charbon, de l'oxyde d'aluminium, du carbonate de calcium ou du sulfate de baryum.

18. Procédé selon la revendication 16, caractérisé en ce que l'alcaloïde du cinchona est la (−)-quinine, la (+)-quinidine, la (+)-cinchonine ou la (−)-cinchonidine.

19. Procédé selon la revendication 16, caractérisé en ce que l'alcaloïde du cinchona est la (−)-conchonidine.

20. Procédé selon la revendication 16, caractérisé en ce que l'on part de l'ester éthylique de l'acide 4-phényl-2-oxo-butyrique.

21. Utilisation des composés de formule I représentée dans la revendication 1 pour la préparation des composés de formule VI:

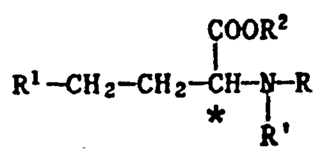

(VI)

caractérisée en ce que l'on alcoyle un composé de formule VII

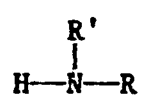

(VII)

sous inversion avec un composé de formule I:

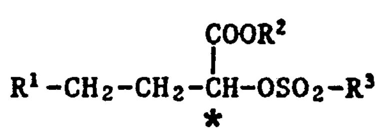

(I)

où $R^1$, $R^2$ et $R^3$ sont définis comme précédemment, le symbole * marque un atome de carbone se présentant, soit dans un nombre prédominant de molécules dans la configuration R, soit dans un nombre

prédominant de molécules dans la configuration R et où R' est l'hydrogène ou un alkyle en $C_1$—$C_7$ et R représente le reste de formule partielle VIII

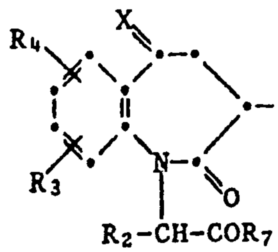

(VIII)

où dans cette formule partielle IX les restes $R_2$, $R_3$, $R_4$ et $R_7$ et X ont les significations définies dans la demande de brevet européen 72 352 où R' est l'hydrogène ou un alkyle en $C_1$—$C_7$ et R représente le reste de formule partielle IX:

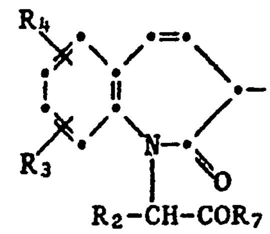

(IX)

où dans cette formule partielle IX les restes $R_2$, $R_3$, $R_4$ et $R_7$ ont les significations définies dans la demande de brevet européen 72 352 où R' représente l'hydrogène et R représente un 1-arylalkyle en $C_1$—$C_7$ où R' et R représentent l'hydrogène.

22. Utilisation des composés de formule I représentée dans la revendication 1 selon la revendication 21 pour la préparation des composés de formule VIa:

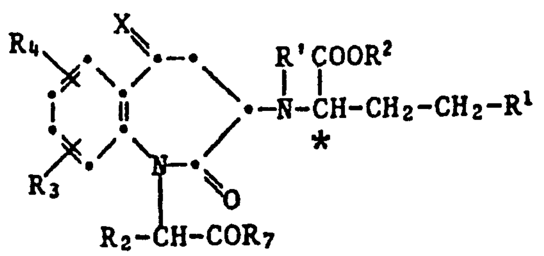

(VIa)

où $R^1$, $R^2$, R', * ainsi que $R_2$, $R_3$, $R_4$, $R_7$ et X sont définis comme dans la revendication 21.

23. Utilisation des composés de formule I représentée dans la revendication 1, selon la revendication 21 pour la préparation des composés de formule VIb

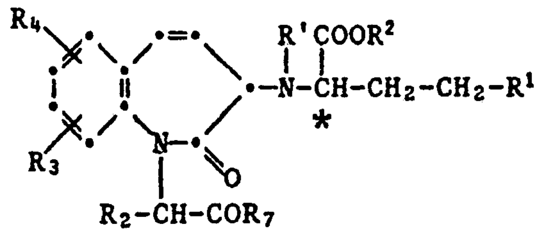

(VIb)

où $R^1$, $R^2$, R', * ainsi que $R_2$, $R_3$, $R_4$ et $R_7$ sont définis comme dans la revendication 21.

24. Utilisation des composés de formule VIb représentée dans la revendication 23 pour la préparation des composés de formule VIa représentée dans la représente 22 où X représente deux atomes d'hydrogène, caractérisée en ce que l'on hydrogénolyse un composé de formule VIb.

25. Utilisation des composés de formule I représentée dans la revendication 1, selon la revendication 21 pour la préparation des composés de formule VIc:

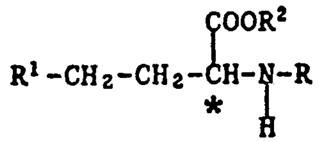

(VIc)

où $R^1$, $R^2$ et * sont définis comme précédemment et où R représente un 1-arylalkyle en $C_1$—$C_7$, par exemple le benzyle, le 1-phényléthyle ou le 1-naphtyléthyle.

26. Utilisation des composés de formule I représentée dans la revendication 1, selon la revendication 21 pour la préparation des composés de formule VId:

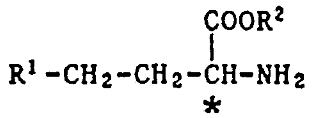

(VId)

où $R^1$, $R^2$ et * sont définis comme précédemment.

27. Utilisation des composés de formule I représentée dans la revendication 1, selon la revendication 21 pour la préparation de la 1-carboxy-méthyle-3S-[(1S-éthoxycarbonyl-3-phényl-propyl)-amino]-2,3,4,5-tétrahydro-1H-[1]-benzazépin-2-one.

**Claims for the Contracting States: BE CH DE FR GB IT LI NL SE**

1. Compounds of formula I

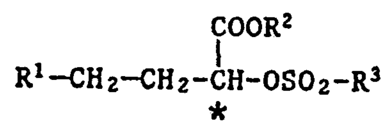

(I)

wherein $R^1$ is $C_5$—$C_6$cycloalkyl which is unsubstituted or substituted by $C_1$—$C_7$alkyl, or is unsubstituted or substituted phenyl, $R^2$ is $C_1$—$C_7$alkyl, $R^3$ is phenyl which is substituted by halogen or by nitro, and the asterisk denotes a carbon atom that is either present in the preponderant number of molecules in the S configuration or in the preponderant number of molecules in the R configuration.

2. Compounds of formula I according to claim 1, wherein $R^2$, $R^3$ and the asterisk are as defined in claim 1 and $R^1$ is phenyl that may be substituted by one or more substituents selected from the group consisting of $C_1$—$C_7$alkyl, hydroxy, $C_1$—$C_7$alkoxy, $C_1$—$C_7$alkanoyloxy, fluorine, $C_1$—$C_7$alkylenedioxy, amino, $C_1$—$C_7$alkylamino, di($C_1$—$C_7$)alkylamino, $C_1$—$C_7$alkanoylamino, carbamoyl, $C_1$—$C_7$alkylcarbamoyl, di($C_1$—$C_7$) alkylcarbamoyl, $C_1$—$C_7$alkanesulfonylamino, sulfamoyl, $C_1$—$C_7$alkylsulfamoyl, di($C_1$—$C_7$)alkylsulfamoyl, $C_1$—$C_7$haloalkyl, $C_1$—$C_7$hydroxyalkyl and $C_1$—$C_7$aminoalkyl.

3. Compounds of formula I according to claim 1, wherein $R^2$, $R^3$ and the asterisk are as defined in claim 1 and $R^1$ is $C_5$—$C_6$cycloalkyl, phenyl or phenyl which is substituted by $C_1$—$C_7$alkyl, hydroxy, $C_1$—$C_7$alkoxy, $C_1$—$C_7$alkanoyloxy, fluorine, trifluoromethyl or by $C_1$—$C_7$alkylenedioxy.

4. Compounds of formula I according to claim 1, wherein $R^1$ is $C_5$—$C_6$cycloalkyl, phenyl or phenyl which is substituted by one of the radicals selected from $C_1$—$C_4$alkyl, hydroxy, $C_1$—$C_4$alkoxy or fluorine, $R^2$ is $C_1$—$C_4$alkyl, $R^3$ is 2-, 3- or 4-nitrophenyl, 2,4-dinitrophenyl or pentafluorophenyl, and the asterisk denotes a carbon atom that is either present in the preponderant number of molecules in the S configuration or in the preponderant number of molecules in the R configuration.

5. Compounds of formula I according to claim 1, wherein $R^1$ is cyclohexyl, phenyl, $C_1$—$C_4$alkylphenyl or $C_1$—$C_4$alkoxyphenyl, $R^2$ is $C_1$—$C_4$alkyl, $R^3$ is 4-nitrophenyl or 2,4-dinitrophenyl, and the asterisk denotes a carbon atom that is present in the preponderant number of molecules in the R configuration.

6. Compounds of formula I according to claim 1, wherein $R^1$ is phenyl, $R^2$ is $C_1$—$C_4$alkyl, $R^3$ is 4-nitrophenyl or 2,4-dinitrophenyl and the asterisk denotes a carbon atom that is present in the preponderant number of molecules in the R configuration.

7. Ethyl (+)-R-2-(4-nitrobenzenesulfonyloxy)-4-phenylbutyrate and ethyl (−)-R-2-(2,4-dinitrobenzenesulfonyloxy)-4-phenyl-butyrate.

8. A process for the preparation of compounds of the formula I as indicated in claim 1, wherein $R^1$, $R^2$, $R^3$ and the asterisk are as defined above, which comprises reacting an α-hydroxy ester of formula II

$$R^1-CH_2-CH_2-\overset{COOR^2}{\underset{*}{CH}}-OH \qquad (II)$$

wherein $R^1$ and $R^2$ are as defined for formula I and the asterisk denotes a carbon atom that is either present in the preponderant number of molecules in the S configuration or in the preponderant number of molecules in the R configuration, with a compound that converts the —OH substituent into the radical of formula $IOSO_2—R^3$, wherein $R^3$ is as defined for formula I.

9. A process according to claim 8, wherein an $R^3$-sulfonic acid anhydride is used.

10. A process according to claim 8, wherein an $R^3$-sulfonyl halide is used.

11. A process according to claim 8, wherein a 4-nitrobenzenesulfonyl halide is used.

12. A process according to claim 8, wherein a 3-nitrobenzenesulfonyl halide is used.

13. A process according to claim 8, wherein a 2,4-dinitrobenzenesulfonyl halide is used.

14. A process according to claim 8, wherein a pentafluorobenzenesulfonyl halide is used.

15. A process according to claim 8, wherein an anhydride of the $R^3—SO_2—O—SO_2—R^3$ type is used.

16. A process for the preparation of compounds of the formula I as indicated in claim 1, wherein $R^1$, $R^2$, $R^3$ and the asterisk are as defined above, which comprises enantio-selectively reducing a compound of formula III

$$R^1-CH_2-CH_2-\overset{COOR^2}{C}=O \qquad (III)$$

wherein $R^1$ and $R^2$ are as defined for formula I, in the presence of a platinum catalyst on a carrier as well as of a cinchona alkaloid, to a compound of formula II as defined above

$$R^1-CH_2-CH_2-\overset{COOR^2}{\underset{*}{CH}}-OH \qquad (II)$$

and reacting the resulting compound of formula II with a compound that converts the —OH substituent into the radical of formula $—OSO_2—R^3$, wherein $R^3$ is as defined for formula I.

17. A process according to claim 16, wherein the carrier is carbon, alumina, calcium carbonate or barium sulfate.

18. A process according to claim 16, wherein the cinchona alkaloid is (−)-quinine, (+)-quinidine, (+)-cinchonine or (−)-cinchonidine.

19. A process according to claim 16, wherein the cinchona alkaloid is (−)-cinchonidine.

20. A process according to claim 16, wherein the starting material is ethyl 4-phenyl-2-oxo-butyrate.

21. use of compounds of formula I as indicated in claim 1 for the preparation of compounds of formula VI

$$R^1-CH_2-CH_2-\overset{COOR^2}{\underset{*}{CH}}-\underset{R'}{N}-R \qquad (VI)$$

which comprises alkylating a compound of formula VII

$$H—\overset{R'}{N}—R \qquad (VII)$$

with inversion, with a compound of formula I

$$R^1-CH_2-CH_2-\overset{COOR^2}{\underset{*}{CH}}-OSO_2-R^3 \qquad (I)$$

wherein $R^1$, $R^2$ and $R^3$ are as defined above, the asterisk denotes a carbon atom that is either present in the

preponderant number of molecules in the S configuration or in the preponderant number of molecules in the R configuration, and R′ is hydrogen or $C_1$—$C_7$alkyl and R is the radical of partial formula VIII

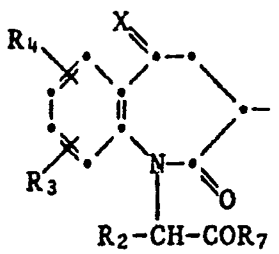

(VIII)

wherein, in this partial formula VIII, the radicals $R_2$, $R_3$, $R_4$, $R_7$ and X are as defined in European patent application 72 352, or wherein R′ is hydrogen or $C_1$—$C_7$alkyl and R is the radical of partial formula IX

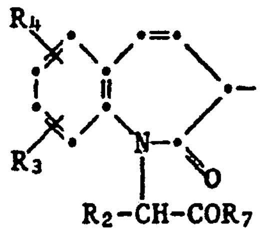

(IX)

wherein, in this partial formula IX, the radicals $R_2$, $R_3$, $R_4$ and $R_7$ are as defined in European patent application 72 352, or wherein R′ is hydrogen and R is 1-aryl-$C_1$—$C_7$alkyl, or wherein R′ and R are hydrogen.

22. Use of compounds of formula I as indicated in claim 1 according to claim 21 for the preparation of compounds of formula VIa.

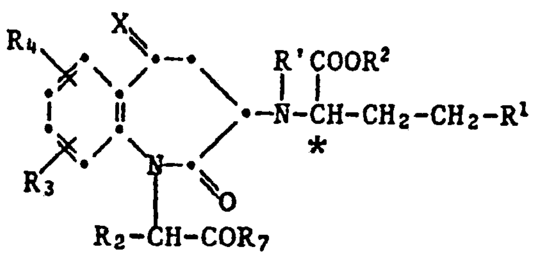

(VIa)

wherein $R^1$, $R^2$, R′, the asterisk and $R_2$, $R_3$, $R_4$, $R_7$ and X are as defined in claim 21.

23. Use of compounds of formula I as indicated in claim 1 according to claim 21 for the preparation of compounds of formula VIb.

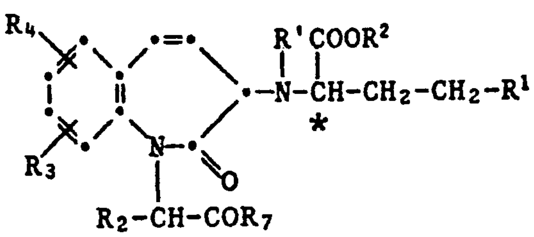

(VIb)

wherein $R^1$, $R^2$, R′, the asterisk and $R_2$, $R_3$, $R_4$ and $R_7$ are as defined in claim 21.

24. Use of compounds of formula VIb as indicated in claim 23 for the preparation of compounds of formula VIa as indicated in claim 22, wherein X denotes two hydrogen atoms, which comprises hydrogenolysing a compound of formula VIb.

25. Use of compounds of formula I as indicated in claim 1 according to claim 21 for the preparation of compounds of formula VIc

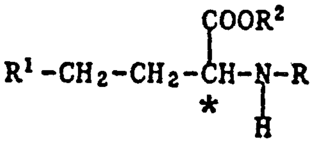

(VIc)

wherein $R^1$, $R^2$ and the asterisk are as defined above and R is 1-aryl-$C_1$—$C_7$alkyl, for example benzyl, 1-phenylethyl or 1-naphthylethyl.

26. Use of compounds of formula I as indicated in claim 1 according to claim 21 for the preparation of compounds of formula VId

$$R^1-CH_2-CH_2-\underset{*}{\overset{\overset{COOR^2}{|}}{C}}H-NH_2 \qquad (VId)$$

wherein $R^1$, $R^2$ and the asterisk are as defined above.

27. Use of compounds of formula I as indicated in claim 1 according to claim 21 for the preparation of 1-carboxymethyl-3S-[(1S-ethoxycarbonyl-3-phenyl-propyl)amino]-2,3,4,5-tetrahydro-1H-[1]-benzazepin-2-one.

28. Ethyl (+)-R-2-(3-nitrobenzenesulfonyloxy)-4-phenylbutyrate.

**Claims for the Contracting State: AT**

1. A process for the preparation of compounds of formula I

$$R^1-CH_2-CH_2-\underset{*}{\overset{\overset{COOR^2}{|}}{C}}H-OSO_2-R^3 \qquad (I)$$

wherein $R^1$ is $C_5$—$C_6$cycloalkyl which is unsubstituted or substituted by $C_1$—$C_7$alkyl, or is unsubstituted or substituted phenyl, $R^2$ is $C_1$—$C_7$alkyl, $R^3$ is phenyl which is substituted by halogen or by nitro, and the asterisk denotes a carbon atom that is either present in the preponderant number of molecules in the S configuration or in the preponderant number of molecules in the R configuration, which comprises reacting an α-hydroxy ester of formula II

$$R^1-CH_2-CH_2-\underset{*}{\overset{\overset{COOR^2}{|}}{C}}H-OH \qquad (II)$$

wherein $R^1$ and $R^2$ are as defined for formula I and the asterisk denotes a carbon atom that is either present in the preponderant number of molecules in the S configuration or in the preponderant number of molecules in the R configuration, with a compound that converts the —OH substituent into the radical of formula —$OSO_2$—$R^3$, wherein $R^3$ is as defined for formula I.

2. A process according to claim 1 for the preparation of compounds of formula I, wherein $R^2$, $R^3$ and the asterisk are as defined in claim 1 and $R^1$ is phenyl that may be substituted by one or more substituents selected from the group consisting of $C_1$—$C_7$alkyl, hydroxy, $C_1$—$C_7$alkoxy, $C_1$—$C_7$alkanoyloxy, fluorine, $C_1$—$C_7$alkylenedioxy, amino, $C_1$—$C_7$alkylamino, di($C_1$—$C_7$)alkylamino, $C_1$—$C_7$alkanoylamino, carbamoyl, $C_1$—$C_7$alkylcarbamoyl, di($C_1$—$C_7$) alkylcarbamoyl, $C_1$—$C_7$alkanesulfonylamino, sulfamoyl, $C_1$—$C_7$alkylsulfamoyl, di($C_1$—$C_7$)alkylsulfamoyl, $C_1$—$C_7$haloalkyl, $C_1$—$C_7$hydroxyalkyl and $C_1$—$C_7$aminoalkyl.

3. A process according to claim 1 for the preparation of compounds of formula I, wherein $R^2$, $R^3$ and the asterisk are as defined in claim 1 and $R^1$ is $C_5$—$C_6$cycloalkyl, phenyl or phenyl which is substituted by $C_1$—$C_7$alkyl, hydroxy, $C_1$—$C_7$alkoxy, $C_1$—$C_7$alkanoyloxy, fluorine, trifluoromethyl or by $C_1$—$C_7$alkylenedioxy.

4. A process according to claim 1 for the preparation of compounds of formula I, wherein $R^1$ is $C_5$—$C_6$cycloalkyl, phenyl or phenyl which is substituted by one of the radicals selected from $C_1$—$C_4$alkyl, hydroxy, $C_1$—$C_4$alkoxy or fluorine, $R^2$ is $C_1$—$C_4$alkyl, $R^3$ is 2-, 3- or 4-nitrophenyl, 2,4-dinitrophenyl or pentafluorophenyl, and the asterisk denotes a carbon atom that is either present in the preponderant number of molecules in the S configuration or in the preponderant number of molecules in the R configuration.

5. A process according to claim 1 for the preparation of compounds of formula I, wherein $R^1$ is cyclohexyl, phenyl, $C_1$—$C_4$alkylphenyl or $C_1$—$C_4$alkoxyphenyl, $R^2$ is $C_1$—$C_4$alkyl, $R^3$ is 4-nitrophenyl or 2,4-dinitrophenyl, and the asterisk denotes a carbon atom that is present in the preponderant number of molecules in the R configuration.

6. A process according to claim 1 for the preparation of compounds of formula I, wherein $R^1$ is phenyl, $R^2$ is $C_1$—$C_4$alkyl, $R^3$ is 4-nitrophenyl or 2,4-dinitrophenyl and the asterisk denotes a carbon atom that is present in the preponderant number of molecules in the R configuration.

7. A process according to claim 1 for the preparation of ethyl (+)-R-2-(4-nitrobenzenesulfonyloxy)-4-phenylbutyrate and ethyl (−)-R-2-(2,4-dinitrobenzenesulfonyloxy)-4-phenyl-butyrate.

8. A process according to claim 1 for the preparation of ethyl (+)-R-2-(3-nitrobenzenesulfonyloxy)-4-phenylbutyrate.

9. A process according to claim 1, wherein an $R^3$-sulfonic acid anhydride is used.

10. A process according to claim 1, wherein an $R^3$-sulfonyl halide is used.

11. A process according to claim 1, wherein a 4-nitrobenzenesulfonyl halide is used.

12. A process according to claim 1, wherein a 3-nitrobenzenesulfonyl halide is used.

13. A process according to claim 1, wherein a 2,4-nitrobenzenesulfonyl halide is used.

14. A process according to claim 1, wherein a pentafluorobenzenesulfonyl halide is used.

15. A process according to claim 1, wherein an anhydride of the $R^3—SO_2—O—SO_2—R^3$ type is used.

16. A process for the preparation of compounds of the formula I as indicated in claim 1, wherein $R^1$, $R^2$, $R^3$ and the asterisk are as defined above, which comprises enantio-selectively reducing a compound of formula III

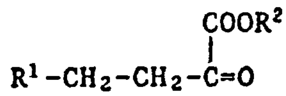

(III)

wherein $R^1$ and $R^2$ are as defined for formula I, in the presence of a platinum catalyst on a carrier as well as of a cinchona alkaloid, to a compound of formula II as defined above

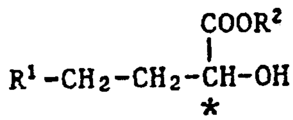

(II)

and reacting the resulting compound of formula II with a compound that converts the —OH substituent into the radical of formula $—OSO_2—R^3$, wherein $R^3$ is as defined for formula I.

17. A process according to claim 16, wherein the carrier is carbon, alumina, calcium carbonate or barium sulfate.

18. A process according to claim 16, wherein the cinchona alkaloid is (−)-quinine, (+)-quinidine, (+)-cinchonine or (−)-cinchonidine.

19. A process according to claim 16, wherein the cinchona alkaloid is (−)-cinchonidine.

20. A process according to claim 16, wherein the starting material is ethyl 4-phenyl-2-oxo-butyrate.

21. Use of compounds of formula I as indicated in claim 1 for the preparation of compounds of formula VI

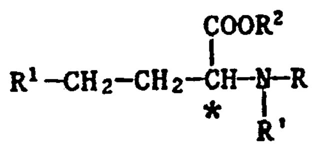

(VI)

which comprises alkylating a compound of formula VII

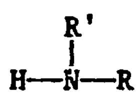

(VII)

with inversion, with a compound of formula I

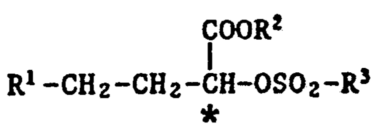

(I)

wherein $R^1$, $R^2$ and $R^3$ are as defined above, the asterisk denotes a carbon atom that is either present in the preponderant number of molecules in the S configuration or in the preponderant number of molecules in the R configuration, and R′ is hydrogen or $C_1—C_7$alkyl and R is the radical of partial formula VIII

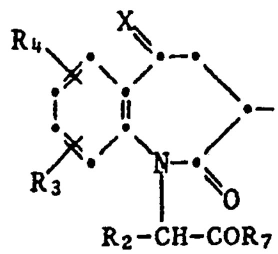

(VIII)

wherein, in this partial formula VIII, the radicals $R_2$, $R_3$, $R_4$, $R_7$ and X are as defined in European patent application 72 352, or wherein R′ is hydrogen or $C_1$—$C_7$alkyl and R is the radical of partial formula IX

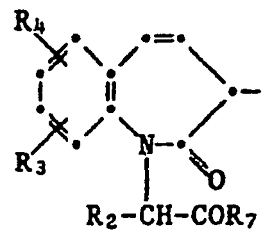

(IX)

wherein, in this partial formula IX, the radicals $R_2$, $R_3$, $R_4$ and $R_7$ are as defined in European patent application 72 352, or wherein R′ is hydrogen and R is 1-aryl-$C_1$—$C_7$alkyl, or wherein R′ and R are hydrogen.

22. Use of compounds of formula I as indicated in claim 1 according to claim 21 for the preparation of compounds of formula VIa.

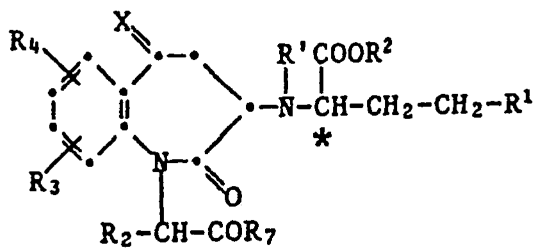

(VIa)

wherein $R^1$, $R^2$, R′, the asterisk and $R_2$, $R_3$, $R_4$, $R_7$ and X are as defined in claim 21.

23. Use of compounds of formula I as indicated in claim 1 according to claim 21 for the preparation of compounds of formula VIb.

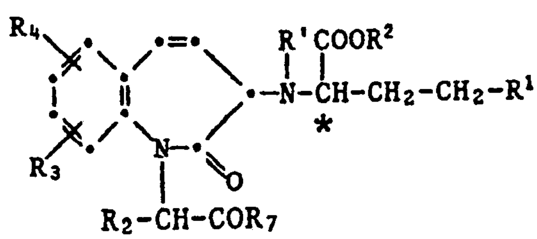

(VIb)

wherein $R^1$, $R^2$, R′, the asterisk and $R_2$, $R_3$, $R_4$ and $R_7$ are as defined in claim 21.

24. Use of compounds of formula VIb as indicated in claim 23 for the preparation of compounds of formula VIa as indicated in claim 22, wherein X denotes two hydrogen atoms, which comprises hydrogenolysing a compound of formula VIb.

25. Use of compounds of formula I as indicated in claim 1 according to claim 21 for the preparation of compounds of formula VIc

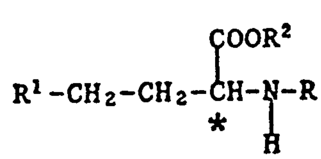

(VIc)

wherein $R^1$, $R^2$ and the asterisk are as defined above and R is 1-aryl-$C_1$—$C_7$alkyl, for example benzyl, 1-phenylethyl or 1-naphthylethyl.

26. Use of compounds of formula I as indicated in claim 1 according to claim 21 for the preparation of compounds of formula VId

$$R^1-CH_2-CH_2-\overset{\overset{\displaystyle COOR^2}{|}}{\underset{*}{C}}H-NH_2 \qquad (VId)$$

wherein $R^1$, $R^2$ and the asterisk are as defined above.

27. Use of compounds of formula I as indicated in claim 1 according to claim 21 for the preparation of 1-carboxymethyl-3S-[(1S-ethoxycarbonyl-3-phenyl-propyl)amino]-2,3,4,5-tetrahydro-1H-[1]-benzazepin-2-one.